# EUROPEAN PATENT SPECIFICATION

(11) **EP 2 655 408 B1**
(45) Date of publication and mention of the grant of the patent: **06.07.2016**
(21) Application number: 11802949.5
(22) Date of filing: 22.12.2011
(51) Int. Cl.: C07K 14/475, A61K 38/18

(54) **ROBO1-FC FUSION PROTEIN FOR USE IN THE TREATMENT OF HEPATOCARCINOMA**
ROBO1-FC-FUSIONSPROTEIN ZUR VERWENDUNG BEI DER BEHANDLUNG VON LEBERKARZINOM
PROTÉINE HYBRIDES ROBO1-FC À UTILISER DANS TRAITEMENT D' HÉPATOCARCINOMES

(30) Priority: 23.12.2010 FR 1061163; 14.10.2011 EP 11306336
(43) Date of publication of application: 30.10.2013
(73) Proprietor: Sanofi, 75013 Paris (FR)
(72) Inventor: DOL-GLEIZES, Frédérique, 75013 Paris (FR); GUEGUEN-DORBES, Geneviève, 75013 Paris (FR)
(74) Representative: Jan, Gaëlle
(86) International application number: PCT/EP2011/073739
(87) International publication number: WO 2012/085178

(56) References cited:
- EP-A1- 1 744 162
- WO-A1-99/20764
- WO-A1-2008/134046
- WO-A1-2011/134420
- WO-A2-03/075860
- LIU ZHE ET AL: "Extracellular Ig domains 1 and 2 of Robo are important for ligand (Slit) binding", MOLECULAR AND CELLULAR NEUROSCIENCES, vol. 26, no. 2, 1 June 2004 (2004-06-01), pages 232-240, XP002605466, SAN DIEGO, US ISSN: 1044-7431, DOI: 10.1016/J.MCN.2004.01.002 [retrieved on 2004-04-16]
- HIVERT BRUNO ET AL: "Robo1 and Robo2 are homophilic binding molecules that promote axonal growth", MOLECULAR AND CELLULAR NEUROSCIENCES, vol. 21, no. 4, 1 December 2002 (2002-12-01), pages 534-545, XP002605467, SAN DIEGO, US ISSN: 1044-7431, DOI: 10.1006/MCNE.2002.1193
- XIAN JIAN ET AL: "Targeted disruption of the 3p12 gene, Dutt1/Robo1, predisposes mice to lung adenocarcinomas and lymphomas with methylation of the gene promoter", CANCER RESEARCH, vol. 64, no. 18, 15 September 2004 (2004-09-15), pages 6432-6437, XP002605471, AMERICAN ASSOCIATION FOR CANCER RESEARCH, US ISSN: 0008-5472
- HANNULA-JOUPPI KATARIINA ET AL: "The axon guidance receptor gene ROBO1 is a candidate gene for developmental dyslexia.", PLOS GENETICS, vol. 1, no. 4, E50, October 2005 (2005-10) , pages 0467-0474, XP002675582, ISSN: 1553-7404
- DALLOL ASHRAF ET AL: "Tumour specific promoter region methylation of the human homologue of the Drosophila Roundabout gene DUTT1 (ROBO1) in human cancers.", ONCOGENE, vol. 21, no. 19, 2 May 2002 (2002-05-02), pages 3020-3028, XP002675583, ISSN: 0950-9232
- GRÖNE JÖRN ET AL: "Robo1/Robo4: differential expression of angiogenic markers in colorectal cancer.", ONCOLOGY REPORTS, vol. 15, no. 6, June 2006 (2006-06), pages 1437-1443, XP002675584, ISSN: 1021-335X
- WANG LI-JING ET AL: "Targeting Slit-Roundabout signaling inhibits tumor angiogenesis in chemical-induced squamous cell carcinogenesis.", CANCER SCIENCE, vol. 99, no. 3, March 2008 (2008-03), pages 510-517, XP002675585, ISSN: 1349-7006
- CLARK K ET AL: "Temporal and spatial expression of two isoforms of the Dutt1/Robo1 gene in mouse development", FEBS LETTERS, vol. 523, no. 1-3, 17 July 2002 (2002-07-17), pages 12-16, XP004371138, ELSEVIER, AMSTERDAM, NL ISSN: 0014-5793, DOI: 10.1016/S0014-5793(02)02904-6
- NURAL HIKMET FEYZA ET AL: "The Slit receptor Robo1 is predominantly expressed via the Dutt1 alternative promoter in pioneer neurons in the embryonic mouse brain and spinal cord", GENE EXPRESSION PATTERNS, vol. 7, no. 8, 1 October 2007 (2007-10-01) , pages 837-845, XP002605468, ELSEVIER ISSN: 1567-133X, DOI: 10.1016/J.MODGEP.2007.07.004
- DATABASE UniProt [Online] 1 January 1998 (1998-01-01), KIDD T ET AL: "RecName: Full=Roundabout homolog 1; AltName: Full=Deleted in U twenty twenty; AltName: Full=H-Robo-1; Flags: Precursor", XP002605469, Database accession no. Q9Y6N7
- MORLOT CECILE ET AL: "Structural insights into the Slit-Robo complex", PROCEEDINGS OF THE NATIONAL ACADEMY OF SCIENCES, vol. 104, no. 38, 18 September 2007 (2007-09-18), pages 14923-14928, XP002605470, NATIONAL ACADEMY OF SCIENCES, WASHINGTON, DC; US ISSN: 0027-8424, DOI: 10.1073//ONAS.0705310104
- WANG B ET AL: "Induction of tumor angiogenesis by Slit-Robo signaling and inhibition of cancer growth by blocking Robo activity", CANCER CELL, vol. 4, no. 1, 1 July 2003 (2003-07-01), pages 19-29, XP002996193, CELL PRESS, US ISSN: 1535-6108, DOI: 10.1016/S1535-6108(03)00164-8

### SEQUENCE LISTING

<110> SANOFI
<120> Robo1-Fc fusion protein for use in the treatment of hepatocarcinoma
<130> FR2010-090 PCT
<160> 24
<170> PatentIn version 3.3
<210> 1
   <211> 660
   <212> DNA
   <213> Homo sapiens
<220>
   <221> CDS
   <222> (1)..(660)
<400> 1
   atg att gcg gag ccc gct cac ttt tac ctg ttt gga tta ata tgt ctc      48
   Met Ile Ala Glu Pro Ala His Phe Tyr Leu Phe Gly Leu Ile Cys Leu
   1               5                   10                  15
   tgt tca ggc tcc cgt ctt cgt cag gaa gat ttt cca cct cgc att gtt      96
   Cys Ser Gly Ser Arg Leu Arg Gln Glu Asp Phe Pro Pro Arg Ile Val
               20                  25                  30
   gaa cac cct tca gac ctg att gtc tca aaa gga gaa cct gca act ttg     144
   Glu His Pro Ser Asp Leu Ile Val Ser Lys Gly Glu Pro Ala Thr Leu
           35                  40                  45
   aac tgc aaa gct gaa ggc cgc ccc aca ccc act att gaa tgg tac aaa     192
   Asn Cys Lys Ala Glu Gly Arg Pro Thr Pro Thr Ile Glu Trp Tyr Lys
       50                  55                  60
   ggg gga gag aga gtg gag aca gac aaa gat gac cct cgc tca cac cga     240
   Gly Gly Glu Arg Val Glu Thr Asp Lys Asp Asp Pro Arg Ser His Arg
   65                  70                  75                  80
   atg ttg ctg ccg agt gga tct tta ttt ttc tta cgt ata gta cat gga     288
   Met Leu Leu Pro Ser Gly Ser Leu Phe Phe Leu Arg Ile Val His Gly
                   85                  90                  95
   cgg aaa agt aga cct gat gaa gga gtc tat gtc tgt gta gca agg aat     336
   Arg Lys Ser Arg Pro Asp Glu Gly Val Tyr Val Cys Val Ala Arg Asn
               100                 105                 110
   tac ctt gga gag gct gtg agc cac aat gca tcg ctg gaa gta gcc ata     384
   Tyr Leu Gly Glu Ala Val Ser His Asn Ala Ser Leu Glu Val Ala Ile
           115                 120                 125
   ctt cgg gat gac ttc aga caa aac cct tcg gat gtc atg gtt gca gta     432
   Leu Arg Asp Asp Phe Arg Gln Asn Pro Ser Asp Val Met Val Ala Val
       130                 135                 140
   gga gag cct gca gta atg gaa tgc caa cct cca cga ggc cat cct gag     480
   Gly Glu Pro Ala Val Met Glu Cys Gln Pro Pro Arg Gly His Pro Glu
   145                 150                 155                 160
   ccc acc att tca tgg aag aaa gat ggc tct cca ctg gat gat aaa gat     528
   Pro Thr Ile Ser Trp Lys Lys Asp Gly Ser Pro Leu Asp Asp Lys Asp
                   165                 170                 175
   gaa aga ata act ata cga gga gga aag ctc atg atc act tac acc cgt     576
   Glu Arg Ile Thr Ile Arg Gly Gly Lys Leu Met Ile Thr Tyr Thr Arg
               180                 185                 190
   aaa agt gac gct ggc aaa tat gtt tgt gtt ggt acc aat atg gtt ggg     624
   Lys Ser Asp Ala Gly Lys Tyr Val Cys Val Gly Thr Asn Met Val Gly
           195                 200                 205
   gaa cgt gag agt gaa gta gcc gag ctg act gtc tta                     660
   Glu Arg Glu Ser Glu Val Ala Glu Leu Thr Val Leu
       210                 215                 220
<210> 2
   <211> 220
   <212> PRT
   <213> Homo sapiens
<400> 2
   Met Ile Ala Glu Pro Ala His Phe Tyr Leu Phe Gly Leu Ile Cys Leu
   1               5                   10                  15
   Cys Ser Gly Ser Arg Leu Arg Gln Glu Asp Phe Pro Pro Arg Ile Val
               20                  25                  30
   Glu His Pro Ser Asp Leu Ile Val Ser Lys Gly Glu Pro Ala Thr Leu
           35                  40                  45
   Asn Cys Lys Ala Glu Gly Arg Pro Thr Pro Thr Ile Glu Trp Tyr Lys
       50                  55                  60
   Gly Gly Glu Arg Val Glu Thr Asp Lys Asp Asp Pro Arg Ser His Arg
   65                  70                  75                  80
   Met Leu Leu Pro Ser Gly Ser Leu Phe Phe Leu Arg Ile Val His Gly
                   85                  90                  95
   Arg Lys Ser Arg Pro Asp Glu Gly Val Tyr Val Cys Val Ala Arg Asn
               100                 105                 110
   Tyr Leu Gly Glu Ala Val Ser His Asn Ala Ser Leu Glu Val Ala Ile
           115                 120                 125
   Leu Arg Asp Asp Phe Arg Gln Asn Pro Ser Asp Val Met Val Ala Val
       130                 135                 140
   Gly Glu Pro Ala Val Met Glu Cys Gln Pro Pro Arg Gly His Pro Glu
   145                 150                 155                 160
   Pro Thr Ile Ser Trp Lys Lys Asp Gly Ser Pro Leu Asp Asp Lys Asp
                   165                 170                 175
   Glu Arg Ile Thr Ile Arg Gly Gly Lys Leu Met Ile Thr Tyr Thr Arg
               180                 185                 190
   Lys Ser Asp Ala Gly Lys Tyr Val Cys Val Gly Thr Asn Met Val Gly
           195                 200                 205
   Glu Arg Glu Ser Glu Val Ala Glu Leu Thr Val Leu
       210                 215                 220
<210> 3
   <211> 1365
   <212> DNA
   <213> Homo sapiens
<220>
   <221> CDS
   <222> (1)..(1365)
<400> 3
   atg att gcg gag ccc gct cac ttt tac ctg ttt gga tta ata tgt ctc      48
   Met Ile Ala Glu Pro Ala His Phe Tyr Leu Phe Gly Leu Ile Cys Leu
   1               5                   10                  15
   tgt tca ggc tcc cgt ctt cgt cag gaa gat ttt cca cct cgc att gtt      96
   Cys Ser Gly Ser Arg Leu Arg Gln Glu Asp Phe Pro Pro Arg Ile Val
               20                  25                  30
   gaa cac cct tca gac ctg att gtc tca aaa gga gaa cct gca act ttg     144
   Glu His Pro Ser Asp Leu Ile Val Ser Lys Gly Glu Pro Ala Thr Leu
           35                  40                  45
   aac tgc aaa gct gaa ggc cgc ccc aca ccc act att gaa tgg tac aaa     192
   Asn Cys Lys Ala Glu Gly Arg Pro Thr Pro Thr Ile Glu Trp Tyr Lys
       50                  55                  60
   ggg gga gag aga gtg gag aca gac aaa gat gac cct cgc tca cac cga     240
   Gly Gly Glu Arg Val Glu Thr Asp Lys Asp Asp Pro Arg Ser His Arg
   65                  70                  75                  80
   atg ttg ctg ccg agt gga tct tta ttt ttc tta cgt ata gta cat gga     288
   Met Leu Leu Pro Ser Gly Ser Leu Phe Phe Leu Arg Ile Val His Gly
                   85                  90                  95
   cgg aaa agt aga cct gat gaa gga gtc tat gtc tgt gta gca agg aat     336
   Arg Lys Ser Arg Pro Asp Glu Gly Val Tyr Val Cys Val Ala Arg Asn
               100                 105                 110
   tac ctt gga gag gct gtg agc cac aat gca tcg ctg gaa gta gcc ata     384
   Tyr Leu Gly Glu Ala Val Ser His Asn Ala Ser Leu Glu Val Ala Ile
           115                 120                 125
   ctt cgg gat gac ttc aga caa aac cct tcg gat gtc atg gtt gca gta     432
   Leu Arg Asp Asp Phe Arg Gln Asn Pro Ser Asp Val Met Val Ala Val
       130                 135                 140
   gga gag cct gca gta atg gaa tgc caa cct cca cga ggc cat cct gag       480
   Gly Glu Pro Ala Val Met Glu Cys Gln Pro Pro Arg Gly His Pro Glu
   145                 150                 155                 160
   ccc acc att tca tgg aag aaa gat ggc tct cca ctg gat gat aaa gat       528
   Pro Thr Ile Ser Trp Lys Lys Asp Gly Ser Pro Leu Asp Asp Lys Asp
                   165                 170                 175
   gaa aga ata act ata cga gga gga aag ctc atg atc act tac acc cgt       576
   Glu Arg Ile Thr Ile Arg Gly Gly Lys Leu Met Ile Thr Tyr Thr Arg
               180                 185                 190
   aaa agt gac gct ggc aaa tat gtt tgt gtt ggt acc aat atg gtt ggg       624
   Lys Ser Asp Ala Gly Lys Tyr Val Cys Val Gly Thr Asn Met Val Gly
           195                 200                 205
   gaa cgt gag agt gaa gta gcc gag ctg act gtc tta gag aga cca tca       672
   Glu Arg Glu Ser Glu Val Ala Glu Leu Thr Val Leu Glu Arg Pro Ser
       210                 215                 220
   ttt gtg gag tcc aag tac ggc cct cct tgc cct ccc tgc cct gcc cct       720
   Phe Val Glu Ser Lys Tyr Gly Pro Pro Cys Pro Pro Cys Pro Ala Pro
   225                 230                 235                 240
   gag ttc gag ggc gga cct agc gtg ttc ctg ttc cct cct aag cct aag       768
   Glu Phe Glu Gly Gly Pro Ser Val Phe Leu Phe Pro Pro Lys Pro Lys
                   245                 250                 255
   gac acc ctg atg atc tcc cgg acc cct gag gtg acc tgt gtg gtg gtg       816
   Asp Thr Leu Met Ile Ser Arg Thr Pro Glu Val Thr Cys Val Val Val
               260                 265                 270
   gac gtg tcc cag gag gac cct gag gtc cag ttc aac tgg tac gtg gac       864
   Asp Val Ser Gln Glu Asp Pro Glu Val Gln Phe Asn Trp Tyr Val Asp
           275                 280                 285
   ggc gtg gag gtg cac aac gcc aag acc aag cct cgg gag gag cag ttc       912
   Gly Val Glu Val His Asn Ala Lys Thr Lys Pro Arg Glu Glu Gln Phe
       290                 295                 300
   aat tcc acc tac cgg gtg gtg tct gtg ctg acc gtg ctg cac cag gac       960
   Asn Ser Thr Tyr Arg Val Val Ser Val Leu Thr Val Leu His Gln Asp
   305                 310                 315                 320
   tgg ctg aac ggc aaa gaa tac aag tgt aag gtc tcc aac aag ggc ctg      1008
   Trp Leu Asn Gly Lys Glu Tyr Lys Cys Lys Val Ser Asn Lys Gly Leu
                   325                 330                 335
   ccc tcc tcc atc gag aaa acc atc tcc aag gcc aag ggc cag cct agg      1056
   Pro Ser Ser Ile Glu Lys Thr Ile Ser Lys Ala Lys Gly Gln Pro Arg
               340                 345                 350
   gag cct cag gtg tac acc ctg cct cct agc cag gaa gag atg acc aag      1104
   Glu Pro Gln Val Tyr Thr Leu Pro Pro Ser Gln Glu Glu Met Thr Lys
           355                 360                 365
   aac cag gtg tcc ctg acc tgt ctg gtg aag ggc ttc tac cct tcc gac      1152
   Asn Gln Val Ser Leu Thr Cys Leu Val Lys Gly Phe Tyr Pro Ser Asp
       370                 375                 380
   atc gcc gtg gag tgg gag tcc aac ggc cag cct gag aac aac tac aag      1200
   Ile Ala Val Glu Trp Glu Ser Asn Gly Gln Pro Glu Asn Asn Tyr Lys
   385                 390                 395                 400
   acc acc cct cct gtg ctg gac tcc gac ggc tcc ttc ttc ctg tac tcc      1248
   Thr Thr Pro Pro Val Leu Asp Ser Asp Gly Ser Phe Phe Leu Tyr Ser
                   405                 410                 415
   agg ctg acc gtg gac aag tcc cgg tgg cag gag ggc aac gtc ttt tcc      1296
   Arg Leu Thr Val Asp Lys Ser Arg Trp Gln Glu Gly Asn Val Phe Ser
               420                 425                 430
   tgc tcc gtg atg cac gag gcc ctg cac aac cac tac acc cag aag tcc      1344
   Cys Ser Val Met His Glu Ala Leu His Asn His Tyr Thr Gln Lys Ser
           435                 440                 445
   ctg tcc ctg tct ctg ggc tga                                          1365
   Leu Ser Leu Ser Leu Gly
       450
<210> 4
   <211> 454
   <212> PRT
   <213> Homo sapiens
<400> 4
   Met Ile Ala Glu Pro Ala His Phe Tyr Leu Phe Gly Leu Ile Cys Leu
   1               5                   10                  15
   Cys Ser Gly Ser Arg Leu Arg Gln Glu Asp Phe Pro Pro Arg Ile Val
               20                  25                  30
   Glu His Pro Ser Asp Leu Ile Val Ser Lys Gly Glu Pro Ala Thr Leu
           35                  40                  45
   Asn Cys Lys Ala Glu Gly Arg Pro Thr Pro Thr Ile Glu Trp Tyr Lys
       50                  55                  60
   Gly Gly Glu Arg Val Glu Thr Asp Lys Asp Asp Pro Arg Ser His Arg
   65                  70                  75                  80
   Met Leu Leu Pro Ser Gly Ser Leu Phe Phe Leu Arg Ile Val His Gly
                   85                  90                  95
   Arg Lys Ser Arg Pro Asp Glu Gly Val Tyr Val Cys Val Ala Arg Asn
               100                 105                 110
   Tyr Leu Gly Glu Ala Val Ser His Asn Ala Ser Leu Glu Val Ala Ile
           115                 120                 125
   Leu Arg Asp Asp Phe Arg Gln Asn Pro Ser Asp Val Met Val Ala Val
       130                 135                 140
   Gly Glu Pro Ala Val Met Glu Cys Gln Pro Pro Arg Gly His Pro Glu
   145 150 155 160
   Pro Thr Ile Ser Trp Lys Lys Asp Gly Ser Pro Leu Asp Asp Lys Asp
   165 170 175
   Glu Arg Ile Thr Ile Arg Gly Gly Lys Leu Met Ile Thr Tyr Thr Arg
   180 185 190
   Lys Ser Asp Ala Gly Lys Tyr Val Cys Val Gly Thr Asn Met Val Gly
   195 200 205
   Glu Arg Glu Ser Glu Val Ala Glu Leu Thr Val Leu Glu Arg Pro Ser
   210 215 220
   Phe Val Glu Ser Lys Tyr Gly Pro Pro Cys Pro Pro Cys Pro Ala Pro
   225 230 235 240
   Glu Phe Glu Gly Gly Pro Ser Val Phe Leu Phe Pro Pro Lys Pro Lys
   245 250 255
   Asp Thr Leu Met Ile Ser Arg Thr Pro Glu Val Thr Cys Val Val Val
   260 265 270
   Asp Val Ser Gln Glu Asp Pro Glu Val Gln Phe Asn Trp Tyr Val Asp
   275 280 285
   Gly Val Glu Val His Asn Ala Lys Thr Lys Pro Arg Glu Glu Gln Phe
   290 295 300
   Asn Ser Thr Tyr Arg Val Val Ser Val Leu Thr Val Leu His Gln Asp
   305 310 315 320
   Trp Leu Asn Gly Lys Glu Tyr Lys Cys Lys Val Ser Asn Lys Gly Leu
   325 330 335
   Pro Ser Ser Ile Glu Lys Thr Ile Ser Lys Ala Lys Gly Gln Pro Arg
   340 345 350
   Glu Pro Gln Val Tyr Thr Leu Pro Pro Ser Gln Glu Glu Met Thr Lys
   355 360 365
   Asn Gln Val Ser Leu Thr Cys Leu Val Lys Gly Phe Tyr Pro Ser Asp
   370 375 380
   Ile Ala Val Glu Trp Glu Ser Asn Gly Gln Pro Glu Asn Asn Tyr Lys
   385 390 395 400
   Thr Thr Pro Pro Val Leu Asp Ser Asp Gly Ser Phe Phe Leu Tyr Ser
                   405                     410                 415
   Arg Leu Thr Val Asp Lys Ser Arg Trp Gln Glu Gly Asn Val Phe Ser
               420                 425                 430
   Cys Ser Val Met His Glu Ala Leu His Asn His Tyr Thr Gln Lys Ser
           435                 440                 445
   Leu Ser Leu Ser Leu Gly
       450
<210> 5
   <211> 1362
   <212> DNA
   <213> Homo sapiens
<220>
   <221> CDS
   <222> (1)..(1362)
<400> 5
   atg att gcg gag ccc gct cac ttt tac ctg ttt gga tta ata tgt ctc      48
   Met Ile Ala Glu Pro Ala His Phe Tyr Leu Phe Gly Leu Ile Cys Leu
   1               5                   10                  15
   tgt tca ggc tcc cgt ctt cgt cag gaa gat ttt cca cct cgc att gtt      96
   Cys Ser Gly Ser Arg Leu Arg Gln Glu Asp Phe Pro Pro Arg Ile Val
               20                  25                  30
   gaa cac cct tca gac ctg att gtc tca aaa gga gaa cct gca act ttg      144
   Glu His Pro Ser Asp Leu Ile Val Ser Lys Gly Glu Pro Ala Thr Leu
           35                  40                  45
   aac tgc aaa gct gaa ggc cgc ccc aca ccc act att gaa tgg tac aaa      192
   Asn Cys Lys Ala Glu Gly Arg Pro Thr Pro Thr Ile Glu Trp Tyr Lys
       50                  55                  60
   ggg gga gag aga gtg gag aca gac aaa gat gac cct cgc tca cac cga      240
   Gly Gly Glu Arg Val Glu Thr Asp Lys Asp Asp Pro Arg Ser His Arg
   65                  70                  75                  80
   atg ttg ctg ccg agt gga tct tta ttt ttc tta cgt ata gta cat gga      288
   Met Leu Leu Pro Ser Gly Ser Leu Phe Phe Leu Arg Ile Val His Gly
                   85                  90                  95
   cgg aaa agt aga cct gat gaa gga gtc tat gtc tgt gta gca agg aat      336
   Arg Lys Ser Arg Pro Asp Glu Gly Val Tyr Val Cys Val Ala Arg Asn
               100                 105                 110
   tac ctt gga gag gct gtg agc cac aat gca tcg ctg gaa gta gcc ata      384
   Tyr Leu Gly Glu Ala Val Ser His Asn Ala Ser Leu Glu Val Ala Ile
           115                 120                 125
   ctt cgg gat gac ttc aga caa aac cct tcg gat gtc atg gtt gca gta      432
   Leu Arg Asp Asp Phe Arg Gln Asn Pro Ser Asp Val Met Val Ala Val
       130                 135                 140
   gga gag cct gca gta atg gaa tgc caa cct cca cga ggc cat cct gag      480
   Gly Glu Pro Ala Val Met Glu Cys Gln Pro Pro Arg Gly His Pro Glu
   145                 150                 155                 160
   ccc acc att tca tgg aag aaa gat ggc tct cca ctg gat gat aaa gat      528
   Pro Thr Ile Ser Trp Lys Lys Asp Gly Ser Pro Leu Asp Asp Lys Asp
                   165                 170                 175
   gaa aga ata act ata cga gga gga aag ctc atg atc act tac acc cgt      576
   Glu Arg Ile Thr Ile Arg Gly Gly Lys Leu Met Ile Thr Tyr Thr Arg
               180                 185                 190
   aaa agt gac gct ggc aaa tat gtt tgt gtt ggt acc aat atg gtt ggg      624
   Lys Ser Asp Ala Gly Lys Tyr Val Cys Val Gly Thr Asn Met Val Gly
           195                 200                 205
   gaa cgt gag agt gaa gta gcc gag ctg act gtc tta gga ggc gga gga      672
   Glu Arg Glu Ser Glu Val Ala Glu Leu Thr Val Leu Gly Gly Gly Gly
       210                 215                 220
   tcc gag tcc aag tac ggc cct cct tgc cct ccc tgc cct gcc cct gag      720
   Ser Glu Ser Lys Tyr Gly Pro Pro Cys Pro Pro Cys Pro Ala Pro Glu
   225                 230                 235                 240
   ttc gag ggc gga cct agc gtg ttc ctg ttc cct cct aag cct aag gac      768
   Phe Glu Gly Gly Pro Ser Val Phe Leu Phe Pro Pro Lys Pro Lys Asp
                   245                 250                 255
   acc ctg atg atc tcc cgg acc cct gag gtg acc tgt gtg gtg gtg gac      816
   Thr Leu Met Ile Ser Arg Thr Pro Glu Val Thr Cys Val Val Val Asp
               260                 265                 270
   gtg tcc cag gag gac cct gag gtc cag ttc aac tgg tac gtg gac ggc      864
   Val Ser Gln Glu Asp Pro Glu Val Gln Phe Asn Trp Tyr Val Asp Gly
           275                 280                 285
   gtg gag gtg cac aac gcc aag acc aag cct cgg gag gag cag ttc aat      912
   Val Glu Val His Asn Ala Lys Thr Lys Pro Arg Glu Glu Gln Phe Asn
       290                 295                 300
   tcc acc tac cgg gtg gtg tct gtg ctg acc gtg ctg cac cag gac tgg      960
   Ser Thr Tyr Arg Val Val Ser Val Leu Thr Val Leu His Gln Asp Trp
   305                 310                 315                 320
   ctg aac ggc aaa gaa tac aag tgt aag gtc tcc aac aag ggc ctg ccc     1008
   Leu Asn Gly Lys Glu Tyr Lys Cys Lys Val Ser Asn Lys Gly Leu Pro
                   325                 330                 335
   tcc tcc atc gag aaa acc atc tcc aag gcc aag ggc cag cct agg gag     1056
   Ser Ser Ile Glu Lys Thr Ile Ser Lys Ala Lys Gly Gln Pro Arg Glu
               340                 345                 350
   cct cag gtg tac acc ctg cct cct agc cag gaa gag atg acc aag aac     1104
   Pro Gln Val Tyr Thr Leu Pro Pro Ser Gln Glu Glu Met Thr Lys Asn
           355                 360                 365
   cag gtg tcc ctg acc tgt ctg gtg aag ggc ttc tac cct tcc gac atc     1152
   Gln Val Ser Leu Thr Cys Leu Val Lys Gly Phe Tyr Pro Ser Asp Ile
       370                 375                 380
   gcc gtg gag tgg gag tcc aac ggc cag cct gag aac aac tac aag acc     1200
   Ala Val Glu Trp Glu Ser Asn Gly Gln Pro Glu Asn Asn Tyr Lys Thr
   385                 390                 395                 400
   acc cct cct gtg ctg gac tcc gac ggc tcc ttc ttc ctg tac tcc agg      1248
   Thr Pro Pro Val Leu Asp Ser Asp Gly Ser Phe Phe Leu Tyr Ser Arg
                   405                 410                 415
   ctg acc gtg gac aag tcc cgg tgg cag gag ggc aac gtc ttt tcc tgc      1296
   Leu Thr Val Asp Lys Ser Arg Trp Gln Glu Gly Asn Val Phe Ser Cys
               420                 425                 430
   tcc gtg atg cac gag gcc ctg cac aac cac tac acc cag aag tcc ctg      1344
   Ser Val Met His Glu Ala Leu His Asn His Tyr Thr Gln Lys Ser Leu
           435                 440                 445
   tcc ctg tct ctg ggc tga                                              1362
   Ser Leu Ser Leu Gly
       450
<210> 6
   <211> 453
   <212> PRT
   <213> Homo sapiens
<400> 6
   Met Ile Ala Glu Pro Ala His Phe Tyr Leu Phe Gly Leu Ile Cys Leu
   1               5                   10                  15
   Cys Ser Gly Ser Arg Leu Arg Gln Glu Asp Phe Pro Pro Arg Ile Val
               20                  25                  30
   Glu His Pro Ser Asp Leu Ile Val Ser Lys Gly Glu Pro Ala Thr Leu
           35                  40                  45
   Asn Cys Lys Ala Glu Gly Arg Pro Thr Pro Thr Ile Glu Trp Tyr Lys
       50                  55                  60
   Gly Gly Glu Arg Val Glu Thr Asp Lys Asp Asp Pro Arg Ser His Arg
   65                  70                  75                  80
   Met Leu Leu Pro Ser Gly Ser Leu Phe Phe Leu Arg Ile Val His Gly
                   85                  90                  95
   Arg Lys Ser Arg Pro Asp Glu Gly Val Tyr Val Cys Val Ala Arg Asn
               100                 105                 110
   Tyr Leu Gly Glu Ala Val Ser His Asn Ala Ser Leu Glu Val Ala Ile
           115                 120                 125
   Leu Arg Asp Asp Phe Arg Gln Asn Pro Ser Asp Val Met Val Ala Val
       130                 135                 140
   Gly Glu Pro Ala Val Met Glu Cys Gln Pro Pro Arg Gly His Pro Glu
   145 150 155 160
   Pro Thr Ile Ser Trp Lys Lys Asp Gly Ser Pro Leu Asp Asp Lys Asp
   165 170 175
   Glu Arg Ile Thr Ile Arg Gly Gly Lys Leu Met Ile Thr Tyr Thr Arg
   180 185 190
   Lys Ser Asp Ala Gly Lys Tyr Val Cys Val Gly Thr Asn Met Val Gly
   195 200 205
   Glu Arg Glu Ser Glu Val Ala Glu Leu Thr Val Leu Gly Gly Gly Gly
   210 215 220
   Ser Glu Ser Lys Tyr Gly Pro Pro Cys Pro Pro Cys Pro Ala Pro Glu
   225 230 235 240
   Phe Glu Gly Gly Pro Ser Val Phe Leu Phe Pro Pro Lys Pro Lys Asp
   245 250 255
   Thr Leu Met Ile Ser Arg Thr Pro Glu Val Thr Cys Val Val Val Asp
   260 265 270
   Val Ser Gln Glu Asp Pro Glu Val Gln Phe Asn Trp Tyr Val Asp Gly
   275 280 285
   Val Glu Val His Asn Ala Lys Thr Lys Pro Arg Glu Glu Gln Phe Asn
   290 295 300
   Ser Thr Tyr Arg Val Val Ser Val Leu Thr Val Leu His Gln Asp Trp
   305 310 315 320
   Leu Asn Gly Lys Glu Tyr Lys Cys Lys Val Ser Asn Lys Gly Leu Pro
   325 330 335
   Ser Ser Ile Glu Lys Thr Ile Ser Lys Ala Lys Gly Gln Pro Arg Glu
   340 345 350
   Pro Gln Val Tyr Thr Leu Pro Pro Ser Gln Glu Glu Met Thr Lys Asn
   355 360 365
   Gln Val Ser Leu Thr Cys Leu Val Lys Gly Phe Tyr Pro Ser Asp Ile
   370 375 380
   Ala Val Glu Trp Glu Ser Asn Gly Gln Pro Glu Asn Asn Tyr Lys Thr
   385 390 395 400
   Thr Pro Pro Val Leu Asp Ser Asp Gly Ser Phe Phe Leu Tyr Ser Arg
   405 410 415
   Leu Thr Val Asp Lys Ser Arg Trp Gln Glu Gly Asn Val Phe Ser Cys
   420 425 430
   Ser Val Met His Glu Ala Leu His Asn His Tyr Thr Gln Lys Ser Leu
   435 440 445
   Ser Leu Ser Leu Gly
   450
<210> 7
   <211> 1365
   <212> DNA
   <213> Homo sapiens
<220>
   <221> CDS
   <222> (1) .. (1365)
<400> 7
   atg att gcg gag ccc gct cac ttt tac ctg ttt gga tta ata tgt ctc        48
   Met Ile Ala Glu Pro Ala His Phe Tyr Leu Phe Gly Leu Ile Cys Leu
   1               5                   10                  15
   tgt tca ggc tcc cgt ctt cgt cag gaa gat ttt cca cct cgc att gtt        96
   Cys Ser Gly Ser Arg Leu Arg Gln Glu Asp Phe Pro Pro Arg Ile Val
               20                  25                  30
   gaa cac cct tca gac cag att gtc tca aaa gga gaa cct gca act ttg       144
   Glu His Pro Ser Asp Gln Ile Val Ser Lys Gly Glu Pro Ala Thr Leu
           35                  40                  45
   aac tgc aaa gct gaa ggc cgc ccc aca ccc act att gaa tgg tac aaa       192
   Asn Cys Lys Ala Glu Gly Arg Pro Thr Pro Thr Ile Glu Trp Tyr Lys
       50                  55                  60
   ggg gga gag aga gtg gag aca gac aaa gat gac cct cgc tca cac cga       240
   Gly Gly Glu Arg Val Glu Thr Asp Lys Asp Asp Pro Arg Ser His Arg
   65                  70                  75                  80
   atg ttg ctg ccg agt gga tct tta tat ttc tta cgt ata gta cat gga       288
   Met Leu Leu Pro Ser Gly Ser Leu Tyr Phe Leu Arg Ile Val His Gly
                   85                  90                  95
   cgg aaa agt aga cct gat gaa gga gtc tat gtc tgt gta gca agg aat       336
   Arg Lys Ser Arg Pro Asp Glu Gly Val Tyr Val Cys Val Ala Arg Asn
               100                 105                 110
   tac ctt gga gag gct gtg agc cac aat gca tcg ctg gaa gta gcc ata       384
   Tyr Leu Gly Glu Ala Val Ser His Asn Ala Ser Leu Glu Val Ala Ile
           115                 120                 125
   ctt cgg gat gac ttc aga caa aac cct tcg gat gtc atg gtt gca gta       432
   Leu Arg Asp Asp Phe Arg Gln Asn Pro Ser Asp Val Met Val Ala Val
       130                 135                 140
   gga gag cct gca gta atg gaa tgc caa cct cca cga ggc cat cct gag       480
   Gly Glu Pro Ala Val Met Glu Cys Gln Pro Pro Arg Gly His Pro Glu
   145                 150                 155                 160
   ccc acc att tca tgg aag aaa gat ggc tct cca ctg gat gat aaa gat       528
   Pro Thr Ile Ser Trp Lys Lys Asp Gly Ser Pro Leu Asp Asp Lys Asp
                   165                 170                 175
   gaa aga ata act ata cga gga gga aag ctc atg atc act tac acc cgt       576
   Glu Arg Ile Thr Ile Arg Gly Gly Lys Leu Met Ile Thr Tyr Thr Arg
               180                 185                 190
   aaa agt gac gct ggc aaa tat gtt tgt gtt ggt acc aat atg gtt ggg       624
   Lys Ser Asp Ala Gly Lys Tyr Val Cys Val Gly Thr Asn Met Val Gly
           195                 200                 205
   gaa cgt gag agt gaa gta gcc gag ctg act gtc tta gag aga cca tca       672
   Glu Arg Glu Ser Glu Val Ala Glu Leu Thr Val Leu Glu Arg Pro Ser
       210                 215                 220
   ttt gtg gag tcc aag tac ggc cct cct tgc cct ccc tgc cct gcc cct       720
   Phe Val Glu Ser Lys Tyr Gly Pro Pro Cys Pro Pro Cys Pro Ala Pro
   225                 230                 235                 240
   gag ttc gag ggc gga cct agc gtg ttc ctg ttc cct cct aag cct aag       768
   Glu Phe Glu Gly Gly Pro Ser Val Phe Leu Phe Pro Pro Lys Pro Lys
                   245                 250                 255
   gac acc ctg atg atc tcc cgg acc cct gag gtg acc tgt gtg gtg gtg       816
   Asp Thr Leu Met Ile Ser Arg Thr Pro Glu Val Thr Cys Val Val Val
               260                 265                 270
   gac gtg tcc cag gag gac cct gag gtc cag ttc aac tgg tac gtg gac       864
   Asp Val Ser Gln Glu Asp Pro Glu Val Gln Phe Asn Trp Tyr Val Asp
           275                 280                 285
   ggc gtg gag gtg cac aac gcc aag acc aag cct cgg gag gag cag ttc       912
   Gly Val Glu Val His Asn Ala Lys Thr Lys Pro Arg Glu Glu Gln Phe
       290                 295                 300
   aat tcc acc tac cgg gtg gtg tct gtg ctg acc gtg ctg cac cag gac       960
   Asn Ser Thr Tyr Arg Val Val Ser Val Leu Thr Val Leu His Gln Asp
   305                 310                 315                 320
   tgg ctg aac ggc aaa gaa tac aag tgt aag gtc tcc aac aag ggc ctg      1008
   Trp Leu Asn Gly Lys Glu Tyr Lys Cys Lys Val Ser Asn Lys Gly Leu
                   325                 330                 335
   ccc tcc tcc atc gag aaa acc atc tcc aag gcc aag ggc cag cct agg      1056
   Pro Ser Ser Ile Glu Lys Thr Ile Ser Lys Ala Lys Gly Gln Pro Arg
               340                 345                 350
   gag cct cag gtg tac acc ctg cct cct agc cag gaa gag atg acc aag      1104
   Glu Pro Gln Val Tyr Thr Leu Pro Pro Ser Gln Glu Glu Met Thr Lys
           355                 360                 365
   aac cag gtg tcc ctg acc tgt ctg gtg aag ggc ttc tac cct tcc gac      1152
   Asn Gln Val Ser Leu Thr Cys Leu Val Lys Gly Phe Tyr Pro Ser Asp
       370                 375                 380
   atc gcc gtg gag tgg gag tcc aac ggc cag cct gag aac aac tac aag      1200
   Ile Ala Val Glu Trp Glu Ser Asn Gly Gln Pro Glu Asn Asn Tyr Lys
   385                 390                 395                 400
   acc acc cct cct gtg ctg gac tcc gac ggc tcc ttc ttc ctg tac tcc      1248
   Thr Thr Pro Pro Val Leu Asp Ser Asp Gly Ser Phe Phe Leu Tyr Ser
                   405                 410                 415
   agg ctg acc gtg gac aag tcc cgg tgg cag gag ggc aac gtc ttt tcc      1296
   Arg Leu Thr Val Asp Lys Ser Arg Trp Gln Glu Gly Asn Val Phe Ser
               420                 425                 430
   tgc tcc gtg atg cac gag gcc ctg cac aac cac tac acc cag aag tcc      1344
   Cys Ser Val Met His Glu Ala Leu His Asn His Tyr Thr Gln Lys Ser
           435                 440                 445
   ctg tcc ctg tct ctg ggc tga                                          1365
   Leu Ser Leu Ser Leu Gly
       450
<210> 8
   <211> 454
   <212> PRT
   <213> Homo sapiens
<400> 8
   Met Ile Ala Glu Pro Ala His Phe Tyr Leu Phe Gly Leu Ile Cys Leu
   1               5                   10                  15
   Cys Ser Gly Ser Arg Leu Arg Gln Glu Asp Phe Pro Pro Arg Ile Val
               20                  25                  30
   Glu His Pro Ser Asp Gln Ile Val Ser Lys Gly Glu Pro Ala Thr Leu
           35                  40                  45
   Asn Cys Lys Ala Glu Gly Arg Pro Thr Pro Thr Ile Glu Trp Tyr Lys
       50                  55                  60
   Gly Gly Glu Arg Val Glu Thr Asp Lys Asp Asp Pro Arg Ser His Arg
   65                  70                  75                  80
   Met Leu Leu Pro Ser Gly Ser Leu Tyr Phe Leu Arg Ile Val His Gly
                   85                  90                  95
   Arg Lys Ser Arg Pro Asp Glu Gly Val Tyr Val Cys Val Ala Arg Asn
               100                 105                 110
   Tyr Leu Gly Glu Ala Val Ser His Asn Ala Ser Leu Glu Val Ala Ile
           115                 120                 125
   Leu Arg Asp Asp Phe Arg Gln Asn Pro Ser Asp Val Met Val Ala Val
       130                 135                 140
   Gly Glu Pro Ala Val Met Glu Cys Gln Pro Pro Arg Gly His Pro Glu
   145                 150                 155                 160
   Pro Thr Ile Ser Trp Lys Lys Asp Gly Ser Pro Leu Asp Asp Lys Asp
   165 170 175
   Glu Arg Ile Thr Ile Arg Gly Gly Lys Leu Met Ile Thr Tyr Thr Arg
   180 185 190
   Lys Ser Asp Ala Gly Lys Tyr Val Cys Val Gly Thr Asn Met Val Gly
   195 200 205
   Glu Arg Glu Ser Glu Val Ala Glu Leu Thr Val Leu Glu Arg Pro Ser
   210 215 220
   Phe Val Glu Ser Lys Tyr Gly Pro Pro Cys Pro Pro Cys Pro Ala Pro
   225 230 235 240
   Glu Phe Glu Gly Gly Pro Ser Val Phe Leu Phe Pro Pro Lys Pro Lys
   245 250 255
   Asp Thr Leu Met Ile Ser Arg Thr Pro Glu Val Thr Cys Val Val Val
   260 265 270
   Asp Val Ser Gln Glu Asp Pro Glu Val Gln Phe Asn Trp Tyr Val Asp
   275 280 285
   Gly Val Glu Val His Asn Ala Lys Thr Lys Pro Arg Glu Glu Gln Phe
   290 295 300
   Asn Ser Thr Tyr Arg Val Val Ser Val Leu Thr Val Leu His Gln Asp
   305 310 315 320
   Trp Leu Asn Gly Lys Glu Tyr Lys Cys Lys Val Ser Asn Lys Gly Leu
   325 330 335
   Pro Ser Ser Ile Glu Lys Thr Ile Ser Lys Ala Lys Gly Gln Pro Arg
   340 345 350
   Glu Pro Gln Val Tyr Thr Leu Pro Pro Ser Gln Glu Glu Met Thr Lys
   355 360 365
   Asn Gln Val Ser Leu Thr Cys Leu Val Lys Gly Phe Tyr Pro Ser Asp
   370 375 380
   Ile Ala Val Glu Trp Glu Ser Asn Gly Gln Pro Glu Asn Asn Tyr Lys
   385 390 395 400
   Thr Thr Pro Pro Val Leu Asp Ser Asp Gly Ser Phe Phe Leu Tyr Ser
   405 410 415
   Arg Leu Thr Val Asp Lys Ser Arg Trp Gln Glu Gly Asn Val Phe Ser
   420 425 430
   Cys Ser Val Met His Glu Ala Leu His Asn His Tyr Thr Gln Lys Ser
   435 440 445
   Leu Ser Leu Ser Leu Gly
   450
<210> 9
   <211> 1365
   <212> DNA
   <213> Homo sapiens
<220>
   <221> CDS
   <222> (1)..(1365)
<400> 9
   atg att gcg gag ccc gct cac ttt tac ctg ttt gga tta ata tgt ctc        48
   Met Ile Ala Glu Pro Ala His Phe Tyr Leu Phe Gly Leu Ile Cys Leu
   1               5                   10                  15
   tgt tca ggc tcc cgt ctt cgt cag gaa gat ttt cca cct cgc att gtt        96
   Cys Ser Gly Ser Arg Leu Arg Gln Glu Asp Phe Pro Pro Arg Ile Val
               20                  25                  30
   gaa cac cct tca gac ctg att gtc tca aaa gga gaa cct gca act ttg       144
   Glu His Pro Ser Asp Leu Ile Val Ser Lys Gly Glu Pro Ala Thr Leu
           35                  40                  45
   aac tgc aaa gct gaa ggc cgc ccc aca ccc act att gaa tgg tac aaa       192
   Asn Cys Lys Ala Glu Gly Arg Pro Thr Pro Thr Ile Glu Trp Tyr Lys
       50                  55                  60
   ggg gga gag aga gtg gag aca gac aaa gat gac cct cgc tca cac cga       240
   Gly Gly Glu Arg Val Glu Thr Asp Lys Asp Asp Pro Arg Ser His Arg
   65                  70                  75                  80
   atg ttg ctg ccg agt gga tct tta ttt ttc tta cgt ata gta cat gga       288
   Met Leu Leu Pro Ser Gly Ser Leu Phe Phe Leu Arg Ile Val His Gly
                   85                  90                  95
   gcc gcc agt aga cct gat gaa gga gtc tat gtc tgt gta gca agg aat       336
   Ala Ala Ser Arg Pro Asp Glu Gly Val Tyr Val Cys Val Ala Arg Asn
               100                 105                 110
   tac ctt gga gag gct gtg agc cac aat gca tcg ctg gaa gta gcc ata       384
   Tyr Leu Gly Glu Ala Val Ser His Asn Ala Ser Leu Glu Val Ala Ile
           115                 120                 125
   ctt cgg gat gac ttc aga caa aac cct tcg gat gtc atg gtt gca gta       432
   Leu Arg Asp Asp Phe Arg Gln Asn Pro Ser Asp Val Met Val Ala Val
       130                 135                 140
   gga gag cct gca gta atg gaa tgc caa cct cca cga ggc cat cct gag       480
   Gly Glu Pro Ala Val Met Glu Cys Gln Pro Pro Arg Gly His Pro Glu
   145                 150                 155                 160
   ccc acc att tca tgg aag aaa gat ggc tct cca ctg gat gat aaa gat      528
   Pro Thr Ile Ser Trp Lys Lys Asp Gly Ser Pro Leu Asp Asp Lys Asp
                   165                 170                 175
   gaa aga ata act ata cga gga gga aag ctc atg atc act tac acc cgt      576
   Glu Arg Ile Thr Ile Arg Gly Gly Lys Leu Met Ile Thr Tyr Thr Arg
               180                 185                 190
   aaa agt gac gct ggc aaa tat gtt tgt gtt ggt acc aat atg gtt ggg      624
   Lys Ser Asp Ala Gly Lys Tyr Val Cys Val Gly Thr Asn Met Val Gly
           195                 200                 205
   gaa cgt gag agt gaa gta gcc gag ctg act gtc tta gag aga cca tca      672
   Glu Arg Glu Ser Glu Val Ala Glu Leu Thr Val Leu Glu Arg Pro Ser
       210                 215                 220
   ttt gtg gag tcc aag tac ggc cct cct tgc cct ccc tgc cct gcc cct      720
   Phe Val Glu Ser Lys Tyr Gly Pro Pro Cys Pro Pro Cys Pro Ala Pro
   225                 230                 235                 240
   gag ttc gag ggc gga cct agc gtg ttc ctg ttc cct cct aag cct aag      768
   Glu Phe Glu Gly Gly Pro Ser Val Phe Leu Phe Pro Pro Lys Pro Lys
                   245                 250                 255
   gac acc ctg atg atc tcc cgg acc cct gag gtg acc tgt gtg gtg gtg      816
   Asp Thr Leu Met Ile Ser Arg Thr Pro Glu Val Thr Cys Val Val Val
               260                 265                 270
   gac gtg tcc cag gag gac cct gag gtc cag ttc aac tgg tac gtg gac      864
   Asp Val Ser Gln Glu Asp Pro Glu Val Gln Phe Asn Trp Tyr Val Asp
           275                 280                 285
   ggc gtg gag gtg cac aac gcc aag acc aag cct cgg gag gag cag ttc      912
   Gly Val Glu Val His Asn Ala Lys Thr Lys Pro Arg Glu Glu Gln Phe
       290                 295                 300
   aat tcc acc tac cgg gtg gtg tct gtg ctg acc gtg ctg cac cag gac      960
   Asn Ser Thr Tyr Arg Val Val Ser Val Leu Thr Val Leu His Gln Asp
   305                 310                 315                 320
   tgg ctg aac ggc aaa gaa tac aag tgt aag gtc tcc aac aag ggc ctg     1008
   Trp Leu Asn Gly Lys Glu Tyr Lys Cys Lys Val Ser Asn Lys Gly Leu
                   325                 330                 335
   ccc tcc tcc atc gag aaa acc atc tcc aag gcc aag ggc cag cct agg     1056
   Pro Ser Ser Ile Glu Lys Thr Ile Ser Lys Ala Lys Gly Gln Pro Arg
               340                 345                 350
   gag cct cag gtg tac acc ctg cct cct agc cag gaa gag atg acc aag     1104
   Glu Pro Gln Val Tyr Thr Leu Pro Pro Ser Gln Glu Glu Met Thr Lys
           355                 360                 365
   aac cag gtg tcc ctg acc tgt ctg gtg aag ggc ttc tac cct tcc gac     1152
   Asn Gln Val Ser Leu Thr Cys Leu Val Lys Gly Phe Tyr Pro Ser Asp
       370                 375                 380
   atc gcc gtg gag tgg gag tcc aac ggc cag cct gag aac aac tac aag     1200
   Ile Ala Val Glu Trp Glu Ser Asn Gly Gln Pro Glu Asn Asn Tyr Lys
   385                 390                 395                 400
   acc acc cct cct gtg ctg gac tcc gac ggc tcc ttc ttc ctg tac tcc      1248
   Thr Thr Pro Pro Val Leu Asp Ser Asp Gly Ser Phe Phe Leu Tyr Ser
                   405                 410                 415
   agg ctg acc gtg gac aag tcc cgg tgg cag gag ggc aac gtc ttt tcc      1296
   Arg Leu Thr Val Asp Lys Ser Arg Trp Gln Glu Gly Asn Val Phe Ser
               420                 425                 430
   tgc tcc gtg atg cac gag gcc ctg cac aac cac tac acc cag aag tcc      1344
   Cys Ser Val Met His Glu Ala Leu His Asn His Tyr Thr Gln Lys Ser
           435                 440                 445
   ctg tcc ctg tct ctg ggc tga                                          1365
   Leu Ser Leu Ser Leu Gly
       450
<210> 10
   <211> 454
   <212> PRT
   <213> Homo sapiens
<400> 10
   Met Ile Ala Glu Pro Ala His Phe Tyr Leu Phe Gly Leu Ile Cys Leu
   1               5                   10                  15
   Cys Ser Gly Ser Arg Leu Arg Gln Glu Asp Phe Pro Pro Arg Ile Val
               20                  25                  30
   Glu His Pro Ser Asp Leu Ile Val Ser Lys Gly Glu Pro Ala Thr Leu
           35                  40                  45
   Asn Cys Lys Ala Glu Gly Arg Pro Thr Pro Thr Ile Glu Trp Tyr Lys
       50                  55                  60
   Gly Gly Glu Arg Val Glu Thr Asp Lys Asp Asp Pro Arg Ser His Arg
   65                  70                  75                  80
   Met Leu Leu Pro Ser Gly Ser Leu Phe Phe Leu Arg Ile Val His Gly
                   85                  90                  95
   Ala Ala Ser Arg Pro Asp Glu Gly Val Tyr Val Cys Val Ala Arg Asn
               100                 105                 110
   Tyr Leu Gly Glu Ala Val Ser His Asn Ala Ser Leu Glu Val Ala Ile
           115                 120                 125
   Leu Arg Asp Asp Phe Arg Gln Asn Pro Ser Asp Val Met Val Ala Val
       130                 135                 140
   Gly Glu Pro Ala Val Met Glu Cys Gln Pro Pro Arg Gly His Pro Glu
   145                 150                 155                 160
   Pro Thr Ile Ser Trp Lys Lys Asp Gly Ser Pro Leu Asp Asp Lys Asp
   165 170 175
   Glu Arg Ile Thr Ile Arg Gly Gly Lys Leu Met Ile Thr Tyr Thr Arg
   180 185 190
   Lys Ser Asp Ala Gly Lys Tyr Val Cys Val Gly Thr Asn Met Val Gly
   195 200 205
   Glu Arg Glu Ser Glu Val Ala Glu Leu Thr Val Leu Glu Arg Pro Ser
   210 215 220
   Phe Val Glu Ser Lys Tyr Gly Pro Pro Cys Pro Pro Cys Pro Ala Pro
   225 230 235 240
   Glu Phe Glu Gly Gly Pro Ser Val Phe Leu Phe Pro Pro Lys Pro Lys
   245 250 255
   Asp Thr Leu Met Ile Ser Arg Thr Pro Glu Val Thr Cys Val Val Val
   260 265 270
   Asp Val Ser Gln Glu Asp Pro Glu Val Gln Phe Asn Trp Tyr Val Asp
   275 280 285
   Gly Val Glu Val His Asn Ala Lys Thr Lys Pro Arg Glu Glu Gln Phe
   290 295 300
   Asn Ser Thr Tyr Arg Val Val Ser Val Leu Thr Val Leu His Gln Asp
   305 310 315 320
   Trp Leu Asn Gly Lys Glu Tyr Lys Cys Lys Val Ser Asn Lys Gly Leu
   325 330 335
   Pro Ser Ser Ile Glu Lys Thr Ile Ser Lys Ala Lys Gly Gln Pro Arg
   340 345 350
   Glu Pro Gln Val Tyr Thr Leu Pro Pro Ser Gln Glu Glu Met Thr Lys
   355 360 365
   Asn Gln Val Ser Leu Thr Cys Leu Val Lys Gly Phe Tyr Pro Ser Asp
   370 375 380
   Ile Ala Val Glu Trp Glu Ser Asn Gly Gln Pro Glu Asn Asn Tyr Lys
   385 390 395 400
   Thr Thr Pro Pro Val Leu Asp Ser Asp Gly Ser Phe Phe Leu Tyr Ser
   405 410 415
   Arg Leu Thr Val Asp Lys Ser Arg Trp Gln Glu Gly Asn Val Phe Ser
               420                 425                 430
   Cys Ser Val Met His Glu Ala Leu His Asn His Tyr Thr Gln Lys Ser
           435                 440                 445
   Leu Ser Leu Ser Leu Gly
       450
<210> 11
   <211> 717
   <212> DNA
   <213> Homo sapiens
<220>
   <221> CDS
   <222> (1) .. (717)
<400> 11
   atg tac agg atg caa ctc ctg tct tgc att gca cta agt ctt gca ctt       48
   Met Tyr Arg Met Gln Leu Leu Ser Cys Ile Ala Leu Ser Leu Ala Leu
   1               5                   10                  15
   gtc acg aat tca ttg cac tgc cct gcc gcc tgt acc tgt agc aac aat       96
   Val Thr Asn Ser Leu His Cys Pro Ala Ala Cys Thr Cys Ser Asn Asn
               20                  25                  30
   atc gta gac tgt cgt ggg aaa ggt ctc act gag atc ccc aca aat ctt      144
   Ile Val Asp Cys Arg Gly Lys Gly Leu Thr Glu Ile Pro Thr Asn Leu
           35                  40                  45
   cca gag acc atc aca gaa ata cgt ttg gaa cag aac aca atc aaa gtc      192
   Pro Glu Thr Ile Thr Glu Ile Arg Leu Glu Gln Asn Thr Ile Lys Val
       50                  55                  60
   atc cct cct gga gct ttc tca cca tat aaa aag ctt aga cga att gac      240
   Ile Pro Pro Gly Ala Phe Ser Pro Tyr Lys Lys Leu Arg Arg Ile Asp
   65                  70                  75                  80
   ctg agc aat aat cag atc tct gaa ctt gca cca gat gct ttc caa gga      288
   Leu Ser Asn Asn Gln Ile Ser Glu Leu Ala Pro Asp Ala Phe Gln Gly
                   85                  90                  95
   cta cgc tct ctg aat tca ctt gtc ctc tat gga aat aaa atc aca gaa      336
   Leu Arg Ser Leu Asn Ser Leu Val Leu Tyr Gly Asn Lys Ile Thr Glu
               100                 105                 110
   ctc ccc aaa agt tta ttt gaa gga ctg ttt tcc tta cag ctc cta tta      384
   Leu Pro Lys Ser Leu Phe Glu Gly Leu Phe Ser Leu Gln Leu Leu Leu
           115                 120                 125
   ttg aat gcc aac aag ata aac tgc ctt cgg gta gat gct ttt cag gat      432
   Leu Asn Ala Asn Lys Ile Asn Cys Leu Arg Val Asp Ala Phe Gln Asp
       130                 135                 140
   ctc cac aac ttg aac ctt ctc tcc cta tat gac aac aag ctt cag acc      480
   Leu His Asn Leu Asn Leu Leu Ser Leu Tyr Asp Asn Lys Leu Gln Thr
   145                 150                 155                 160
   atc gcc aag ggg acc ttt tca cct ctt cgg gcc att caa act atg cat      528
   Ile Ala Lys Gly Thr Phe Ser Pro Leu Arg Ala Ile Gln Thr Met His
                   165                 170                 175
   ttg gcc cag aac ccc ttt att tgt gac tgc cat ctc aag tgg cta gcg      576
   Leu Ala Gln Asn Pro Phe Ile Cys Asp Cys His Leu Lys Trp Leu Ala
               180                 185                 190
   gat tat ctc cat acc aac ccg att gag acc agt ggt gcc cgt tgc acc      624
   Asp Tyr Leu His Thr Asn Pro Ile Glu Thr Ser Gly Ala Arg Cys Thr
           195                 200                 205
   agc ccc cgc cgc ctg gca aac aaa aga att gga cag atc aaa agc aag      672
   Ser Pro Arg Arg Leu Ala Asn Lys Arg Ile Gly Gln Ile Lys Ser Lys
       210                 215                 220
   aaa ttc cgt tgt tca ggt agc gct cat cac cat cat cat cac tga          717
   Lys Phe Arg Cys Ser Gly Ser Ala His His His His His His
   225                 230                 235
<210> 12
   <211> 238
   <212> PRT
   <213> Homo sapiens
<400> 12
   Met Tyr Arg Met Gln Leu Leu Ser Cys Ile Ala Leu Ser Leu Ala Leu
   1               5                   10                  15
   Val Thr Asn Ser Leu His Cys Pro Ala Ala Cys Thr Cys Ser Asn Asn
               20                  25                  30
   Ile Val Asp Cys Arg Gly Lys Gly Leu Thr Glu Ile Pro Thr Asn Leu
           35                  40                  45
   Pro Glu Thr Ile Thr Glu Ile Arg Leu Glu Gln Asn Thr Ile Lys Val
       50                  55                  60
   Ile Pro Pro Gly Ala Phe Ser Pro Tyr Lys Lys Leu Arg Arg Ile Asp
   65                  70                  75                  80
   Leu Ser Asn Asn Gln Ile Ser Glu Leu Ala Pro Asp Ala Phe Gln Gly
                   85                  90                  95
   Leu Arg Ser Leu Asn Ser Leu Val Leu Tyr Gly Asn Lys Ile Thr Glu
               100                 105                 110
   Leu Pro Lys Ser Leu Phe Glu Gly Leu Phe Ser Leu Gln Leu Leu Leu
           115                 120                 125
   Leu Asn Ala Asn Lys Ile Asn Cys Leu Arg Val Asp Ala Phe Gln Asp
       130                 135                 140
   Leu His Asn Leu Asn Leu Leu Ser Leu Tyr Asp Asn Lys Leu Gln Thr
   145                 150                 155                 160
   Ile Ala Lys Gly Thr Phe Ser Pro Leu Arg Ala Ile Gln Thr Met His
                   165                 170                 175
   Leu Ala Gln Asn Pro Phe Ile Cys Asp Cys His Leu Lys Trp Leu Ala
               180                 185                 190
   Asp Tyr Leu His Thr Asn Pro Ile Glu Thr Ser Gly Ala Arg Cys Thr
           195                 200                 205
   Ser Pro Arg Arg Leu Ala Asn Lys Arg Ile Gly Gln Ile Lys Ser Lys
       210                 215                 220
   Lys Phe Arg Cys Ser Gly Ser Ala His His His His His His
   225                 230                 235
<210> 13
   <211> 1467
   <212> DNA
   <213> Homo sapiens
<220>
   <221> CDS
   <222> (1)..(1467)
<400> 13
   atg cgc ggc gtt ggc tgg cag atg ctg tcc ctg tcg ctg ggg tta gtg      48
   Met Arg Gly Val Gly Trp Gln Met Leu Ser Leu Ser Leu Gly Leu Val
   1               5                   10                  15
   ctg gcg atc ctg aac aag gtg gca ccg cag gcg tgc ccg gcg cag tgc      96
   Leu Ala Ile Leu Asn Lys Val Ala Pro Gln Ala Cys Pro Ala Gln Cys
               20                  25                  30
   tct tgc tcg ggc agc aca gtg gac tgt cac ggg ctg gcg ctg cgc agc     144
   Ser Cys Ser Gly Ser Thr Val Asp Cys His Gly Leu Ala Leu Arg Ser
           35                  40                  45
   gtg ccc agg aat atc ccc cgc aac acc gag aga ctg gat tta aat gga     192
   Val Pro Arg Asn Ile Pro Arg Asn Thr Glu Arg Leu Asp Leu Asn Gly
       50                  55                  60
   aat aac atc aca aga att acg aag aca gat ttt gct ggt ctt aga cat     240
   Asn Asn Ile Thr Arg Ile Thr Lys Thr Asp Phe Ala Gly Leu Arg His
   65                  70                  75                  80
   cta aga gtt ctt cag ctt atg gag aat aag att agc acc att gaa aga     288
   Leu Arg Val Leu Gln Leu Met Glu Asn Lys Ile Ser Thr Ile Glu Arg
                   85                  90                  95
   gga gca ttc cag gat ctt aaa gaa cta gag aga ctg cgt tta aac aga     336
   Gly Ala Phe Gln Asp Leu Lys Glu Leu Glu Arg Leu Arg Leu Asn Arg
               100                 105                 110
   aat cac ctt cag ctg ttt cct gag ttg ctg ttt ctt ggg act gcg aag      384
   Asn His Leu Gln Leu Phe Pro Glu Leu Leu Phe Leu Gly Thr Ala Lys
           115                 120                 125
   cta tac agg ctt gat ctc agt gaa aac caa att cag gca atc cca agg      432
   Leu Tyr Arg Leu Asp Leu Ser Glu Asn Gln Ile Gln Ala Ile Pro Arg
       130                 135                 140
   aaa gct ttc cgt ggg gca gtt gac ata aaa aat ttg caa ctg gat tac      480
   Lys Ala Phe Arg Gly Ala Val Asp Ile Lys Asn Leu Gln Leu Asp Tyr
   145                 150                 155                 160
   aac cag atc agc tgt att gaa gat ggg gca ttc agg gct ctc cgg gac      528
   Asn Gln Ile Ser Cys Ile Glu Asp Gly Ala Phe Arg Ala Leu Arg Asp
                   165                 170                 175
   ctg gaa gtg ctc act ctc aac aat aac aac att act aga ctt tct gtg      576
   Leu Glu Val Leu Thr Leu Asn Asn Asn Asn Ile Thr Arg Leu Ser Val
               180                 185                 190
   gca agt ttc aac cat atg cct aaa ctt agg act ttt cga ctg cat tca      624
   Ala Ser Phe Asn His Met Pro Lys Leu Arg Thr Phe Arg Leu His Ser
           195                 200                 205
   aac aac ctg tat tgt gac tgc cac ctg gcc tgg ctc tcc gac tgg ctt      672
   Asn Asn Leu Tyr Cys Asp Cys His Leu Ala Trp Leu Ser Asp Trp Leu
       210                 215                 220
   cgc caa agg cct cgg gtt ggt ctg tac act cag tgt atg ggc ccc tcc      720
   Arg Gln Arg Pro Arg Val Gly Leu Tyr Thr Gln Cys Met Gly Pro Ser
   225                 230                 235                 240
   cac ctg aga ggc cat aat gta gcc gag gtt caa aaa cga gaa ttt gtc      768
   His Leu Arg Gly His Asn Val Ala Glu Val Gln Lys Arg Glu Phe Val
                   245                 250                 255
   tgc agt ggt cac cag tca ttt atg gct cct tct tgt agt gtt ttg cac      816
   Cys Ser Gly His Gln Ser Phe Met Ala Pro Ser Cys Ser Val Leu His
               260                 265                 270
   tgc cct gcc gcc tgt acc tgt agc aac aat atc gta gac tgt cgt ggg      864
   Cys Pro Ala Ala Cys Thr Cys Ser Asn Asn Ile Val Asp Cys Arg Gly
           275                 280                 285
   aaa ggt ctc act gag atc ccc aca aat ctt cca gag acc atc aca gaa      912
   Lys Gly Leu Thr Glu Ile Pro Thr Asn Leu Pro Glu Thr Ile Thr Glu
       290                 295                 300
   ata cgt ttg gaa cag aac aca atc aaa gtc atc cct cct gga gct ttc      960
   Ile Arg Leu Glu Gln Asn Thr Ile Lys Val Ile Pro Pro Gly Ala Phe
   305                 310                 315                 320
   tca cca tat aaa aag ctt aga cga att gac ctg agc aat aat cag atc     1008
   Ser Pro Tyr Lys Lys Leu Arg Arg Ile Asp Leu Ser Asn Asn Gln Ile
                   325                 330                 335
   tct gaa ctt gca cca gat gct ttc caa gga cta cgc tct ctg aat tca     1056
   Ser Glu Leu Ala Pro Asp Ala Phe Gln Gly Leu Arg Ser Leu Asn Ser
               340                 345                 350
   ctt gtc ctc tat gga aat aaa atc aca gaa ctc ccc aaa agt tta ttt      1104
   Leu Val Leu Tyr Gly Asn Lys Ile Thr Glu Leu Pro Lys Ser Leu Phe
           355                 360                 365
   gaa gga ctg ttt tcc tta cag ctc cta tta ttg aat gcc aac aag ata      1152
   Glu Gly Leu Phe Ser Leu Gln Leu Leu Leu Leu Asn Ala Asn Lys Ile
       370                 375                 380
   aac tgc ctt cgg gta gat gct ttt cag gat ctc cac aac ttg aac ctt      1200
   Asn Cys Leu Arg Val Asp Ala Phe Gln Asp Leu His Asn Leu Asn Leu
   385                 390                 395                 400
   ctc tcc cta tat gac aac aag ctt cag acc atc gcc aag ggg acc ttt      1248
   Leu Ser Leu Tyr Asp Asn Lys Leu Gln Thr Ile Ala Lys Gly Thr Phe
                   405                 410                 415
   tca cct ctt cgg gcc att caa act atg cat ttg gcc cag aac ccc ttt      1296
   Ser Pro Leu Arg Ala Ile Gln Thr Met His Leu Ala Gln Asn Pro Phe
               420                 425                 430
   att tgt gac tgc cat ctc aag tgg cta gcg gat tat ctc cat acc aac      1344
   Ile Cys Asp Cys His Leu Lys Trp Leu Ala Asp Tyr Leu His Thr Asn
           435                 440                 445
   ccg att gag acc agt ggt gcc cgt tgc acc agc ccc cgc cgc ctg gca      1392
   Pro Ile Glu Thr Ser Gly Ala Arg Cys Thr Ser Pro Arg Arg Leu Ala
       450                 455                 460
   aac aaa aga att gga cag atc aaa agc aag aaa ttc cgt tgt tca ggt      1440
   Asn Lys Arg Ile Gly Gln Ile Lys Ser Lys Lys Phe Arg Cys Ser Gly
   465                 470                 475                 480
   agc gct cat cac cat cat cat cac tga                                  1467
   Ser Ala His His His His His His
                   485
<210> 14
   <211> 488
   <212> PRT
   <213> Homo sapiens
<400> 14
   Met Arg Gly Val Gly Trp Gln Met Leu Ser Leu Ser Leu Gly Leu Val
   1               5                   10                  15
   Leu Ala Ile Leu Asn Lys Val Ala Pro Gln Ala Cys Pro Ala Gln Cys
               20                  25                  30
   Ser Cys Ser Gly Ser Thr Val Asp Cys His Gly Leu Ala Leu Arg Ser
           35                  40                  45
   Val Pro Arg Asn Ile Pro Arg Asn Thr Glu Arg Leu Asp Leu Asn Gly
       50                  55                  60
   Asn Asn Ile Thr Arg Ile Thr Lys Thr Asp Phe Ala Gly Leu Arg His
   65                  70                  75                  80
   Leu Arg Val Leu Gln Leu Met Glu Asn Lys Ile Ser Thr Ile Glu Arg
                   85                  90                  95
   Gly Ala Phe Gln Asp Leu Lys Glu Leu Glu Arg Leu Arg Leu Asn Arg
               100                 105                 110
   Asn His Leu Gln Leu Phe Pro Glu Leu Leu Phe Leu Gly Thr Ala Lys
           115                 120                 125
   Leu Tyr Arg Leu Asp Leu Ser Glu Asn Gln Ile Gln Ala Ile Pro Arg
       130                 135                 140
   Lys Ala Phe Arg Gly Ala Val Asp Ile Lys Asn Leu Gln Leu Asp Tyr
   145                 150                 155                 160
   Asn Gln Ile Ser Cys Ile Glu Asp Gly Ala Phe Arg Ala Leu Arg Asp
                   165                 170                 175
   Leu Glu Val Leu Thr Leu Asn Asn Asn Asn Ile Thr Arg Leu Ser Val
               180                 185                 190
   Ala Ser Phe Asn His Met Pro Lys Leu Arg Thr Phe Arg Leu His Ser
           195                 200                 205
   Asn Asn Leu Tyr Cys Asp Cys His Leu Ala Trp Leu Ser Asp Trp Leu
       210                 215                 220
   Arg Gln Arg Pro Arg Val Gly Leu Tyr Thr Gln Cys Met Gly Pro Ser
   225                 230                 235                 240
   His Leu Arg Gly His Asn Val Ala Glu Val Gln Lys Arg Glu Phe Val
                   245                 250                 255
   Cys Ser Gly His Gln Ser Phe Met Ala Pro Ser Cys Ser Val Leu His
               260                 265                 270
   Cys Pro Ala Ala Cys Thr Cys Ser Asn Asn Ile Val Asp Cys Arg Gly
           275                 280                 285
   Lys Gly Leu Thr Glu Ile Pro Thr Asn Leu Pro Glu Thr Ile Thr Glu
       290                 295                 300
   Ile Arg Leu Glu Gln Asn Thr Ile Lys Val Ile Pro Pro Gly Ala Phe
   305                 310                 315                 320
   Ser Pro Tyr Lys Lys Leu Arg Arg Ile Asp Leu Ser Asn Asn Gln Ile
                   325                 330                 335
   Ser Glu Leu Ala Pro Asp Ala Phe Gln Gly Leu Arg Ser Leu Asn Ser
               340                 345                 350
   Leu Val Leu Tyr Gly Asn Lys Ile Thr Glu Leu Pro Lys Ser Leu Phe
           355                 360                 365
   Glu Gly Leu Phe Ser Leu Gln Leu Leu Leu Leu Asn Ala Asn Lys Ile
       370                 375                 380
   Asn Cys Leu Arg Val Asp Ala Phe Gln Asp Leu His Asn Leu Asn Leu
   385                 390                 395                 400
   Leu Ser Leu Tyr Asp Asn Lys Leu Gln Thr Ile Ala Lys Gly Thr Phe
                   405                 410                 415
   Ser Pro Leu Arg Ala Ile Gln Thr Met His Leu Ala Gln Asn Pro Phe
               420                 425                 430
   Ile Cys Asp Cys His Leu Lys Trp Leu Ala Asp Tyr Leu His Thr Asn
           435                 440                 445
   Pro Ile Glu Thr Ser Gly Ala Arg Cys Thr Ser Pro Arg Arg Leu Ala
       450                 455                 460
   Asn Lys Arg Ile Gly Gln Ile Lys Ser Lys Lys Phe Arg Cys Ser Gly
   465                 470                 475                 480
   Ser Ala His His His His His His
                   485
<210> 15
   <211> 3390
   <212> DNA
   <213> Homo sapiens
<220>
   <221> CDS
   <222> (1)..(3390)
<400> 15
   atg cgc ggc gtt ggc tgg cag atg ctg tcc ctg tcg ctg ggg tta gtg      48
   Met Arg Gly Val Gly Trp Gln Met Leu Ser Leu Ser Leu Gly Leu Val
   1               5                   10                  15
   ctg gcg atc ctg aac aag gtg gca ccg cag gcg tgc ccg gcg cag tgc      96
   Leu Ala Ile Leu Asn Lys Val Ala Pro Gln Ala Cys Pro Ala Gln Cys
               20                  25                  30
   tct tgc tcg ggc agc aca gtg gac tgt cac ggg ctg gcg ctg cgc agc     144
   Ser Cys Ser Gly Ser Thr Val Asp Cys His Gly Leu Ala Leu Arg Ser
   35 40 45
   gtg ccc agg aat atc ccc cgc aac acc gag aga ctg gat tta aat gga 192
   Val Pro Arg Asn Ile Pro Arg Asn Thr Glu Arg Leu Asp Leu Asn Gly
   50 55 60
   aat aac atc aca aga att acg aag aca gat ttt gct ggt ctt aga cat 240
   Asn Asn Ile Thr Arg Ile Thr Lys Thr Asp Phe Ala Gly Leu Arg His
   65 70 75 80
   cta aga gtt ctt cag ctt atg gag aat aag att agc acc att gaa aga 288
   Leu Arg Val Leu Gln Leu Met Glu Asn Lys Ile Ser Thr Ile Glu Arg
   85 90 95
   gga gca ttc cag gat ctt aaa gaa cta gag aga ctg cgt tta aac aga 336
   Gly Ala Phe Gln Asp Leu Lys Glu Leu Glu Arg Leu Arg Leu Asn Arg
   100 105 110
   aat cac ctt cag ctg ttt cct gag ttg ctg ttt ctt ggg act gcg aag 384
   Asn His Leu Gln Leu Phe Pro Glu Leu Leu Phe Leu Gly Thr Ala Lys
   115 120 125
   cta tac agg ctt gat ctc agt gaa aac caa att cag gca atc cca agg 432
   Leu Tyr Arg Leu Asp Leu Ser Glu Asn Gln Ile Gln Ala Ile Pro Arg
   130 135 140
   aaa gct ttc cgt ggg gca gtt gac ata aaa aat ttg caa ctg gat tac 480
   Lys Ala Phe Arg Gly Ala Val Asp Ile Lys Asn Leu Gln Leu Asp Tyr
   145 150 155 160
   aac cag atc agc tgt att gaa gat ggg gca ttc agg gct ctc cgg gac 528
   Asn Gln Ile Ser Cys Ile Glu Asp Gly Ala Phe Arg Ala Leu Arg Asp
   165 170 175
   ctg gaa gtg ctc act ctc aac aat aac aac att act aga ctt tct gtg 576
   Leu Glu Val Leu Thr Leu Asn Asn Asn Asn Ile Thr Arg Leu Ser Val
   180 185 190
   gca agt ttc aac cat atg cct aaa ctt agg act ttt cga ctg cat tca 624
   Ala Ser Phe Asn His Met Pro Lys Leu Arg Thr Phe Arg Leu His Ser
   195 200 205
   aac aac ctg tat tgt gac tgc cac ctg gcc tgg ctc tcc gac tgg ctt 672
   Asn Asn Leu Tyr Cys Asp Cys His Leu Ala Trp Leu Ser Asp Trp Leu
   210 215 220
   cgc caa agg cct cgg gtt ggt ctg tac act cag tgt atg ggc ccc tcc 720
   Arg Gln Arg Pro Arg Val Gly Leu Tyr Thr Gln Cys Met Gly Pro Ser
   225 230 235 240
   cac ctg aga ggc cat aat gta gcc gag gtt caa aaa cga gaa ttt gtc 768
   His Leu Arg Gly His Asn Val Ala Glu Val Gln Lys Arg Glu Phe Val
   245 250 255
   tgc agt ggt cac cag tca ttt atg gct cct tct tgt agt gtt ttg cac 816
   Cys Ser Gly His Gln Ser Phe Met Ala Pro Ser Cys Ser Val Leu His
   260 265 270
   tgc cct gcc gcc tgt acc tgt agc aac aat atc gta gac tgt cgt ggg 864
   Cys Pro Ala Ala Cys Thr Cys Ser Asn Asn Ile Val Asp Cys Arg Gly
   275 280 285
   aaa ggt ctc act gag atc ccc aca aat ctt cca gag acc atc aca gaa 912
   Lys Gly Leu Thr Glu Ile Pro Thr Asn Leu Pro Glu Thr Ile Thr Glu
       290                 295                 300
   ata cgt ttg gaa cag aac aca atc aaa gtc atc cct cct gga gct ttc      960
   Ile Arg Leu Glu Gln Asn Thr Ile Lys Val Ile Pro Pro Gly Ala Phe
   305                 310                 315                 320
   tca cca tat aaa aag ctt aga cga att gac ctg agc aat aat cag atc     1008
   Ser Pro Tyr Lys Lys Leu Arg Arg Ile Asp Leu Ser Asn Asn Gln Ile
                   325                 330                 335
   tct gaa ctt gca cca gat gct ttc caa gga cta cgc tct ctg aat tca     1056
   Ser Glu Leu Ala Pro Asp Ala Phe Gln Gly Leu Arg Ser Leu Asn Ser
               340                 345                 350
   ctt gtc ctc tat gga aat aaa atc aca gaa ctc ccc aaa agt tta ttt     1104
   Leu Val Leu Tyr Gly Asn Lys Ile Thr Glu Leu Pro Lys Ser Leu Phe
           355                 360                 365
   gaa gga ctg ttt tcc tta cag ctc cta tta ttg aat gcc aac aag ata     1152
   Glu Gly Leu Phe Ser Leu Gln Leu Leu Leu Leu Asn Ala Asn Lys Ile
       370                 375                 380
   aac tgc ctt cgg gta gat gct ttt cag gat ctc cac aac ttg aac ctt     1200
   Asn Cys Leu Arg Val Asp Ala Phe Gln Asp Leu His Asn Leu Asn Leu
   385                 390                 395                 400
   ctc tcc cta tat gac aac aag ctt cag acc atc gcc aag ggg acc ttt     1248
   Leu Ser Leu Tyr Asp Asn Lys Leu Gln Thr Ile Ala Lys Gly Thr Phe
                   405                 410                 415
   tca cct ctt cgg gcc att caa act atg cat ttg gcc cag aac ccc ttt     1296
   Ser Pro Leu Arg Ala Ile Gln Thr Met His Leu Ala Gln Asn Pro Phe
               420                 425                 430
   att tgt gac tgc cat ctc aag tgg cta gcg gat tat ctc cat acc aac     1344
   Ile Cys Asp Cys His Leu Lys Trp Leu Ala Asp Tyr Leu His Thr Asn
           435                 440                 445
   ccg att gag acc agt ggt gcc cgt tgc acc agc ccc cgc cgc ctg gca     1392
   Pro Ile Glu Thr Ser Gly Ala Arg Cys Thr Ser Pro Arg Arg Leu Ala
       450                 455                 460
   aac aaa aga att gga cag atc aaa agc aag aaa ttc cgt tgt tca gct     1440
   Asn Lys Arg Ile Gly Gln Ile Lys Ser Lys Lys Phe Arg Cys Ser Ala
   465                 470                 475                 480
   aaa gaa cag tat ttc att cca ggt aca gaa gat tat cga tca aaa tta     1488
   Lys Glu Gln Tyr Phe Ile Pro Gly Thr Glu Asp Tyr Arg Ser Lys Leu
                   485                 490                 495
   agt gga gac tgc ttt gcg gat ctg gct tgc cct gaa aag tgt cgc tgt     1536
   Ser Gly Asp Cys Phe Ala Asp Leu Ala Cys Pro Glu Lys Cys Arg Cys
               500                 505                 510
   gaa gga acc aca gta gat tgc tct aat caa aag ctc aac aaa atc ccg     1584
   Glu Gly Thr Thr Val Asp Cys Ser Asn Gln Lys Leu Asn Lys Ile Pro
           515                 520                 525
   gag cac att ccc cag tac act gca gag ttg cgt ctc aat aat aat gaa     1632
   Glu His Ile Pro Gln Tyr Thr Ala Glu Leu Arg Leu Asn Asn Asn Glu
       530                 535                 540
   ttt acc gtg ttg gaa gcc aca gga atc ttt aag aaa ctt cct caa tta      1680
   Phe Thr Val Leu Glu Ala Thr Gly Ile Phe Lys Lys Leu Pro Gln Leu
   545                 550                 555                 560
   cgt aaa ata aac ttt agc aac aat aag atc aca gat att gag gag gga      1728
   Arg Lys Ile Asn Phe Ser Asn Asn Lys Ile Thr Asp Ile Glu Glu Gly
                   565                 570                 575
   gca ttt gaa gga gca tct ggt gta aat gaa ata ctt ctt acg agt aat      1776
   Ala Phe Glu Gly Ala Ser Gly Val Asn Glu Ile Leu Leu Thr Ser Asn
               580                 585                 590
   cgt ttg gaa aat gtg cag cat aag atg ttc aag gga ttg gaa agc ctc      1824
   Arg Leu Glu Asn Val Gln His Lys Met Phe Lys Gly Leu Glu Ser Leu
           595                 600                 605
   aaa act ttg atg ttg aga agc aat cga ata acc tgt gtg ggg aat gac      1872
   Lys Thr Leu Met Leu Arg Ser Asn Arg Ile Thr Cys Val Gly Asn Asp
       610                 615                 620
   agt ttc ata gga ctc agt tct gtg cgt ttg ctt tct ttg tat gat aat      1920
   Ser Phe Ile Gly Leu Ser Ser Val Arg Leu Leu Ser Leu Tyr Asp Asn
   625                 630                 635                 640
   caa att act aca gtt gca cca ggg gca ttt gat act ctc cat tct tta      1968
   Gln Ile Thr Thr Val Ala Pro Gly Ala Phe Asp Thr Leu His Ser Leu
                   645                 650                 655
   tct act cta aac ctc ttg gcc aat cct ttt aac tgt aac tgc tac ctg      2016
   Ser Thr Leu Asn Leu Leu Ala Asn Pro Phe Asn Cys Asn Cys Tyr Leu
               660                 665                 670
   gct tgg ttg gga gag tgg ctg aga aag aag aga att gtc acg gga aat      2064
   Ala Trp Leu Gly Glu Trp Leu Arg Lys Lys Arg Ile Val Thr Gly Asn
           675                 680                 685
   cct aga tgt caa aaa cca tac ttc ctg aaa gaa ata ccc atc cag gat      2112
   Pro Arg Cys Gln Lys Pro Tyr Phe Leu Lys Glu Ile Pro Ile Gln Asp
       690                 695                 700
   gtg gcc att cag gac ttc act tgt gat gac gga aat gat gac aat agt      2160
   Val Ala Ile Gln Asp Phe Thr Cys Asp Asp Gly Asn Asp Asp Asn Ser
   705                 710                 715                 720
   tgc tcc cca ctt tct cgc tgt cct act gaa tgt act tgc ttg gat aca      2208
   Cys Ser Pro Leu Ser Arg Cys Pro Thr Glu Cys Thr Cys Leu Asp Thr
                   725                 730                 735
   gtc gtc cga tgt agc aac aag ggt ttg aag gtc ttg ccg aaa ggt att      2256
   Val Val Arg Cys Ser Asn Lys Gly Leu Lys Val Leu Pro Lys Gly Ile
               740                 745                 750
   cca aga gat gtc aca gag ttg tat ctg gat gga aac caa ttt aca ctg      2304
   Pro Arg Asp Val Thr Glu Leu Tyr Leu Asp Gly Asn Gln Phe Thr Leu
           755                 760                 765
   gtt ccc aag gaa ctc tcc aac tac aaa cat tta aca ctt ata gac tta      2352
   Val Pro Lys Glu Leu Ser Asn Tyr Lys His Leu Thr Leu Ile Asp Leu
       770                 775                 780
   agt aac aac aga ata agc acg ctt tct aat cag agc ttc agc aac atg      2400
   Ser Asn Asn Arg Ile Ser Thr Leu Ser Asn Gln Ser Phe Ser Asn Met
   785                 790                 795                 800
   acc cag ctc ctc acc tta att ctt agt tac aac cgt ctg aga tgt att      2448
   Thr Gln Leu Leu Thr Leu Ile Leu Ser Tyr Asn Arg Leu Arg Cys Ile
                   805                 810                 815
   cct cct cgc acc ttt gat gga tta aag tct ctt cga tta ctt tct cta      2496
   Pro Pro Arg Thr Phe Asp Gly Leu Lys Ser Leu Arg Leu Leu Ser Leu
               820                 825                 830
   cat gga aat gac att tct gtt gtg cct gaa ggt gct ttc aat gat ctt      2544
   His Gly Asn Asp Ile Ser Val Val Pro Glu Gly Ala Phe Asn Asp Leu
           835                 840                 845
   tct gca tta tca cat cta gca att gga gcc aac cct ctt tac tgt gat      2592
   Ser Ala Leu Ser His Leu Ala Ile Gly Ala Asn Pro Leu Tyr Cys Asp
       850                 855                 860
   tgt aac atg cag tgg tta tcc gac tgg gtg aag tcg gaa tat aag gag      2640
   Cys Asn Met Gln Trp Leu Ser Asp Trp Val Lys Ser Glu Tyr Lys Glu
   865                 870                 875                 880
   cct gga att gct cgt tgt gct ggt cct gga gaa atg gca gat aaa ctt      2688
   Pro Gly Ile Ala Arg Cys Ala Gly Pro Gly Glu Met Ala Asp Lys Leu
                   885                 890                 895
   tta ctc aca act ccc tcc aaa aaa ttt acc tgt caa ggt cct gtg gat      2736
   Leu Leu Thr Thr Pro Ser Lys Lys Phe Thr Cys Gln Gly Pro Val Asp
               900                 905                 910
   gtc aat att cta gct aag tgt aac ccc tgc cta tca aat ccg tgt aaa      2784
   Val Asn Ile Leu Ala Lys Cys Asn Pro Cys Leu Ser Asn Pro Cys Lys
           915                 920                 925
   aat gat ggc aca tgt aat agt gat cca gtt gac ttt tac cga tgc acc      2832
   Asn Asp Gly Thr Cys Asn Ser Asp Pro Val Asp Phe Tyr Arg Cys Thr
       930                 935                 940
   tgt cca tat ggt ttc aag ggg cag gac tgt gat gtc cca att cat gcc      2880
   Cys Pro Tyr Gly Phe Lys Gly Gln Asp Cys Asp Val Pro Ile His Ala
   945                 950                 955                 960
   tgc atc agt aac cca tgt aaa cat gga gga act tgc cac tta aag gaa      2928
   Cys Ile Ser Asn Pro Cys Lys His Gly Gly Thr Cys His Leu Lys Glu
                   965                 970                 975
   gga gaa gaa gat gga ttc tgg tgt att tgt gct gat gga ttt gaa gga      2976
   Gly Glu Glu Asp Gly Phe Trp Cys Ile Cys Ala Asp Gly Phe Glu Gly
               980                 985                 990
   gaa aat tgt gaa gtc aac gtt gat  gat tgt gaa gat aat  gac tgt gaa    3024
   Glu Asn Cys Glu Val Asn Val Asp  Asp Cys Glu Asp Asn  Asp Cys Glu
           995                 1000                1005
   aat aat  tct aca tgt gtc gat  ggc att aat aac tac  aca tgc ctt       3069
   Asn Asn  Ser Thr Cys Val Asp  Gly Ile Asn Asn Tyr  Thr Cys Leu
       1010                1015                1020
   tgc cca  cct gag tat aca ggt  gag ttg tgt gag gag  aag ctg gac       3114
   Cys Pro  Pro Glu Tyr Thr Gly  Glu Leu Cys Glu Glu  Lys Leu Asp
       1025                1030                1035
   ttc tgt  gcc cag gac ctg aac  ccc tgc cag cac gat  tca aag tgc       3159
   Phe Cys  Ala Gln Asp Leu Asn  Pro Cys Gln His Asp  Ser Lys Cys
       1040                1045                1050
   atc cta  act cca aag gga ttc  aaa tgt gac tgc aca  cca ggg tac     3204
   Ile Leu  Thr Pro Lys Gly Phe  Lys Cys Asp Cys Thr  Pro Gly Tyr
       1055                1060                1065
   gta ggt  gaa cac tgc gac atc  gat ttt gac gac tgc  caa gac aac     3249
   Val Gly  Glu His Cys Asp Ile  Asp Phe Asp Asp Cys  Gln Asp Asn
       1070                1075                1080
   aag tgt  aaa aac gga gcc cac  tgc aca gat gca gtg  aac ggc tat     3294
   Lys Cys  Lys Asn Gly Ala His  Cys Thr Asp Ala Val  Asn Gly Tyr
       1085                1090                1095
   acg tgc  ata tgc ccc gaa ggt  tac agt ggc ttg ttc  tgt gag ttt     3339
   Thr Cys  Ile Cys Pro Glu Gly  Tyr Ser Gly Leu Phe  Cys Glu Phe
       1100                1105                1110
   tct cca  ccc atg gtc ctc cct  cgt agc gct cat cac  cat cat cat     3384
   Ser Pro  Pro Met Val Leu Pro  Arg Ser Ala His His  His His His
       1115                1120                1125
   cac tga                                                            3390
   His
<210> 16
   <211> 1129
   <212> PRT
   <213> Homo sapiens
<400> 16
   Met Arg Gly Val Gly Trp Gln Met Leu Ser Leu Ser Leu Gly Leu Val
   1               5                   10                  15
   Leu Ala Ile Leu Asn Lys Val Ala Pro Gln Ala Cys Pro Ala Gln Cys
               20                  25                  30
   Ser Cys Ser Gly Ser Thr Val Asp Cys His Gly Leu Ala Leu Arg Ser
           35                  40                  45
   Val Pro Arg Asn Ile Pro Arg Asn Thr Glu Arg Leu Asp Leu Asn Gly
       50                  55                  60
   Asn Asn Ile Thr Arg Ile Thr Lys Thr Asp Phe Ala Gly Leu Arg His
   65                  70                  75                  80
   Leu Arg Val Leu Gln Leu Met Glu Asn Lys Ile Ser Thr Ile Glu Arg
                   85                  90                  95
   Gly Ala Phe Gln Asp Leu Lys Glu Leu Glu Arg Leu Arg Leu Asn Arg
               100                 105                 110
   Asn His Leu Gln Leu Phe Pro Glu Leu Leu Phe Leu Gly Thr Ala Lys
   115 120 125
   Leu Tyr Arg Leu Asp Leu Ser Glu Asn Gln Ile Gln Ala Ile Pro Arg
   130 135 140
   Lys Ala Phe Arg Gly Ala Val Asp Ile Lys Asn Leu Gln Leu Asp Tyr
   145 150 155 160
   Asn Gln Ile Ser Cys Ile Glu Asp Gly Ala Phe Arg Ala Leu Arg Asp
   165 170 175
   Leu Glu Val Leu Thr Leu Asn Asn Asn Asn Ile Thr Arg Leu Ser Val
   180 185 190
   Ala Ser Phe Asn His Met Pro Lys Leu Arg Thr Phe Arg Leu His Ser
   195 200 205
   Asn Asn Leu Tyr Cys Asp Cys His Leu Ala Trp Leu Ser Asp Trp Leu
   210 215 220
   Arg Gln Arg Pro Arg Val Gly Leu Tyr Thr Gln Cys Met Gly Pro Ser
   225 230 235 240
   His Leu Arg Gly His Asn Val Ala Glu Val Gln Lys Arg Glu Phe Val
   245 250 255
   Cys Ser Gly His Gln Ser Phe Met Ala Pro Ser Cys Ser Val Leu His
   260 265 270
   Cys Pro Ala Ala Cys Thr Cys Ser Asn Asn Ile Val Asp Cys Arg Gly
   275 280 285
   Lys Gly Leu Thr Glu Ile Pro Thr Asn Leu Pro Glu Thr Ile Thr Glu
   290 295 300
   Ile Arg Leu Glu Gln Asn Thr Ile Lys Val Ile Pro Pro Gly Ala Phe
   305 310 315 320
   Ser Pro Tyr Lys Lys Leu Arg Arg Ile Asp Leu Ser Asn Asn Gln Ile
   325 330 335
   Ser Glu Leu Ala Pro Asp Ala Phe Gln Gly Leu Arg Ser Leu Asn Ser
   340 345 350
   Leu Val Leu Tyr Gly Asn Lys Ile Thr Glu Leu Pro Lys Ser Leu Phe
   355 360 365
   Glu Gly Leu Phe Ser Leu Gln Leu Leu Leu Leu Asn Ala Asn Lys Ile
   370 375 380
   Asn Cys Leu Arg Val Asp Ala Phe Gln Asp Leu His Asn Leu Asn Leu
   385 390 395 400
   Leu Ser Leu Tyr Asp Asn Lys Leu Gln Thr Ile Ala Lys Gly Thr Phe
   405 410 415
   Ser Pro Leu Arg Ala Ile Gln Thr Met His Leu Ala Gln Asn Pro Phe
   420 425 430
   Ile Cys Asp Cys His Leu Lys Trp Leu Ala Asp Tyr Leu His Thr Asn
   435 440 445
   Pro Ile Glu Thr Ser Gly Ala Arg Cys Thr Ser Pro Arg Arg Leu Ala
   450 455 460
   Asn Lys Arg Ile Gly Gln Ile Lys Ser Lys Lys Phe Arg Cys Ser Ala
   465 470 475 480
   Lys Glu Gln Tyr Phe Ile Pro Gly Thr Glu Asp Tyr Arg Ser Lys Leu
   485 490 495
   Ser Gly Asp Cys Phe Ala Asp Leu Ala Cys Pro Glu Lys Cys Arg Cys
   500 505 510
   Glu Gly Thr Thr Val Asp Cys Ser Asn Gln Lys Leu Asn Lys Ile Pro
   515 520 525
   Glu His Ile Pro Gln Tyr Thr Ala Glu Leu Arg Leu Asn Asn Asn Glu
   530 535 540
   Phe Thr Val Leu Glu Ala Thr Gly Ile Phe Lys Lys Leu Pro Gln Leu
   545 550 555 560
   Arg Lys Ile Asn Phe Ser Asn Asn Lys Ile Thr Asp Ile Glu Glu Gly
   565 570 575
   Ala Phe Glu Gly Ala Ser Gly Val Asn Glu Ile Leu Leu Thr Ser Asn
   580 585 590
   Arg Leu Glu Asn Val Gln His Lys Met Phe Lys Gly Leu Glu Ser Leu
   595 600 605
   Lys Thr Leu Met Leu Arg Ser Asn Arg Ile Thr Cys Val Gly Asn Asp
   610 615 620
   Ser Phe Ile Gly Leu Ser Ser Val Arg Leu Leu Ser Leu Tyr Asp Asn
   625                 630                 635                 640
   Gln Ile Thr Thr Val Ala Pro Gly Ala Phe Asp Thr Leu His Ser Leu
                   645                 650                 655
   Ser Thr Leu Asn Leu Leu Ala Asn Pro Phe Asn Cys Asn Cys Tyr Leu
               660                 665                 670
   Ala Trp Leu Gly Glu Trp Leu Arg Lys Lys Arg Ile Val Thr Gly Asn
           675                 680                 685
   Pro Arg Cys Gln Lys Pro Tyr Phe Leu Lys Glu Ile Pro Ile Gln Asp
       690                 695                 700
   Val Ala Ile Gln Asp Phe Thr Cys Asp Asp Gly Asn Asp Asp Asn Ser
   705                 710                 715                 720
   Cys Ser Pro Leu Ser Arg Cys Pro Thr Glu Cys Thr Cys Leu Asp Thr
                   725                 730                 735
   Val Val Arg Cys Ser Asn Lys Gly Leu Lys Val Leu Pro Lys Gly Ile
               740                 745                 750
   Pro Arg Asp Val Thr Glu Leu Tyr Leu Asp Gly Asn Gln Phe Thr Leu
           755                 760                 765
   Val Pro Lys Glu Leu Ser Asn Tyr Lys His Leu Thr Leu Ile Asp Leu
       770                 775                 780
   Ser Asn Asn Arg Ile Ser Thr Leu Ser Asn Gln Ser Phe Ser Asn Met
   785                 790                 795                 800
   Thr Gln Leu Leu Thr Leu Ile Leu Ser Tyr Asn Arg Leu Arg Cys Ile
                   805                 810                 815
   Pro Pro Arg Thr Phe Asp Gly Leu Lys Ser Leu Arg Leu Leu Ser Leu
               820                 825                 830
   His Gly Asn Asp Ile Ser Val Val Pro Glu Gly Ala Phe Asn Asp Leu
           835                 840                 845
   Ser Ala Leu Ser His Leu Ala Ile Gly Ala Asn Pro Leu Tyr Cys Asp
       850                 855                 860
   Cys Asn Met Gln Trp Leu Ser Asp Trp Val Lys Ser Glu Tyr Lys Glu
   865                 870                 875                 880
   Pro Gly Ile Ala Arg Cys Ala Gly Pro Gly Glu Met Ala Asp Lys Leu
   885 890 895
   Leu Leu Thr Thr Pro Ser Lys Lys Phe Thr Cys Gln Gly Pro Val Asp
   900 905 910
   Val Asn Ile Leu Ala Lys Cys Asn Pro Cys Leu Ser Asn Pro Cys Lys
   915 920 925
   Asn Asp Gly Thr Cys Asn Ser Asp Pro Val Asp Phe Tyr Arg Cys Thr
   930 935 940
   Cys Pro Tyr Gly Phe Lys Gly Gln Asp Cys Asp Val Pro Ile His Ala
   945 950 955 960
   Cys Ile Ser Asn Pro Cys Lys His Gly Gly Thr Cys His Leu Lys Glu
   965 970 975
   Gly Glu Glu Asp Gly Phe Trp Cys Ile Cys Ala Asp Gly Phe Glu Gly
   980 985 990
   Glu Asn Cys Glu Val Asn Val Asp Asp Cys Glu Asp Asn Asp Cys Glu
   995 1000 1005
   Asn Asn Ser Thr Cys Val Asp Gly Ile Asn Asn Tyr Thr Cys Leu
   1010 1015 1020
   Cys Pro Pro Glu Tyr Thr Gly Glu Leu Cys Glu Glu Lys Leu Asp
   1025 1030 1035
   Phe Cys Ala Gln Asp Leu Asn Pro Cys Gln His Asp Ser Lys Cys
   1040 1045 1050
   Ile Leu Thr Pro Lys Gly Phe Lys Cys Asp Cys Thr Pro Gly Tyr
   1055 1060 1065
   Val Gly Glu His Cys Asp Ile Asp Phe Asp Asp Cys Gln Asp Asn
   1070 1075 1080
   Lys Cys Lys Asn Gly Ala His Cys Thr Asp Ala Val Asn Gly Tyr
   1085 1090 1095
   Thr Cys Ile Cys Pro Glu Gly Tyr Ser Gly Leu Phe Cys Glu Phe
   1100 1105 1110
   Ser Pro Pro Met Val Leu Pro Arg Ser Ala His His His His His
   1115 1120 1125
   His
<210> 17
   <211> 717
   <212> DNA
   <213> Mus musculus
<220>
   <221> CDS
   <222> (1) .. (717)
<400> 17
   atg tac agg atg caa ctc ctg tct tgc att gca cta agt ctt gca ctt        48
   Met Tyr Arg Met Gln Leu Leu Ser Cys Ile Ala Leu Ser Leu Ala Leu
   1               5                   10                  15
   gtc acg aat tca ttg cac tgc cct gcc gcc tgt acc tgt agc aac aat        96
   Val Thr Asn Ser Leu His Cys Pro Ala Ala Cys Thr Cys Ser Asn Asn
               20                  25                  30
   atc gta gac tgt cgt ggg aaa ggt ctc act gag atc ccc aca aat ctt       144
   Ile Val Asp Cys Arg Gly Lys Gly Leu Thr Glu Ile Pro Thr Asn Leu
           35                  40                  45
   cca gag acc atc aca gaa ata cgt ttg gaa cag aac agc atc aga gtc       192
   Pro Glu Thr Ile Thr Glu Ile Arg Leu Glu Gln Asn Ser Ile Arg Val
       50                  55                  60
   atc cct cct gga gct ttc tca cca tat aaa aag ctt aga cga ctg gac       240
   Ile Pro Pro Gly Ala Phe Ser Pro Tyr Lys Lys Leu Arg Arg Leu Asp
   65                  70                  75                  80
   ctg agc aat aat cag atc tct gaa ctt gca cca gat gct ttc caa gga       288
   Leu Ser Asn Asn Gln Ile Ser Glu Leu Ala Pro Asp Ala Phe Gln Gly
                   85                  90                  95
   cta cgc tct ctg aat tca ctt gtc ctc tat gga aat aaa atc aca gaa       336
   Leu Arg Ser Leu Asn Ser Leu Val Leu Tyr Gly Asn Lys Ile Thr Glu
               100                 105                 110
   ctc ccc aaa agt tta ttt gaa gga ctg ttt tcc tta cag ctc cta tta       384
   Leu Pro Lys Ser Leu Phe Glu Gly Leu Phe Ser Leu Gln Leu Leu Leu
           115                 120                 125
   ttg aat gcc aac aag ata aac tgc ctt cgg gta gat gct ttt cag gat       432
   Leu Asn Ala Asn Lys Ile Asn Cys Leu Arg Val Asp Ala Phe Gln Asp
       130                 135                 140
   ctc cac aac ttg aac ctt ctc tcc cta tat gac aac aag ctt cag acc       480
   Leu His Asn Leu Asn Leu Leu Ser Leu Tyr Asp Asn Lys Leu Gln Thr
   145                 150                 155                 160
   gtc gcc aag ggg acc ttt tca gct ctt cgg gcc att caa act atg cat       528
   Val Ala Lys Gly Thr Phe Ser Ala Leu Arg Ala Ile Gln Thr Met His
                   165                 170                 175
   ttg gcc cag aac ccc ttt att tgt gac tgc cat ctc aag tgg cta gcg       576
   Leu Ala Gln Asn Pro Phe Ile Cys Asp Cys His Leu Lys Trp Leu Ala
               180                 185                 190
   gat tat ctc cat acc aac ccg att gag acc agt ggt gcc cgt tgc acc       624
   Asp Tyr Leu His Thr Asn Pro Ile Glu Thr Ser Gly Ala Arg Cys Thr
           195                 200                 205
   agc ccc cgc cgc ctg gca aac aaa aga att gga cag atc aaa agc aag       672
   Ser Pro Arg Arg Leu Ala Asn Lys Arg Ile Gly Gln Ile Lys Ser Lys
       210                 215                 220
   aaa ttc cgt tgt tca ggt agc gct cat cac cat cat cat cac tga           717
   Lys Phe Arg Cys Ser Gly Ser Ala His His His His His His
   225                 230                 235
<210> 18
   <211> 238
   <212> PRT
   <213> Mus musculus
<400> 18
   Met Tyr Arg Met Gln Leu Leu Ser Cys Ile Ala Leu Ser Leu Ala Leu
   1               5                   10                  15
   Val Thr Asn Ser Leu His Cys Pro Ala Ala Cys Thr Cys Ser Asn Asn
               20                  25                  30
   Ile Val Asp Cys Arg Gly Lys Gly Leu Thr Glu Ile Pro Thr Asn Leu
           35                  40                  45
   Pro Glu Thr Ile Thr Glu Ile Arg Leu Glu Gln Asn Ser Ile Arg Val
       50                  55                  60
   Ile Pro Pro Gly Ala Phe Ser Pro Tyr Lys Lys Leu Arg Arg Leu Asp
   65                  70                  75                  80
   Leu Ser Asn Asn Gln Ile Ser Glu Leu Ala Pro Asp Ala Phe Gln Gly
                   85                  90                  95
   Leu Arg Ser Leu Asn Ser Leu Val Leu Tyr Gly Asn Lys Ile Thr Glu
               100                 105                 110
   Leu Pro Lys Ser Leu Phe Glu Gly Leu Phe Ser Leu Gln Leu Leu Leu
           115                 120                 125
   Leu Asn Ala Asn Lys Ile Asn Cys Leu Arg Val Asp Ala Phe Gln Asp
       130                 135                 140
   Leu His Asn Leu Asn Leu Leu Ser Leu Tyr Asp Asn Lys Leu Gln Thr
   145                 150                 155                 160
   Val Ala Lys Gly Thr Phe Ser Ala Leu Arg Ala Ile Gln Thr Met His
                   165                 170                 175
   Leu Ala Gln Asn Pro Phe Ile Cys Asp Cys His Leu Lys Trp Leu Ala
               180                 185                 190
   Asp Tyr Leu His Thr Asn Pro Ile Glu Thr Ser Gly Ala Arg Cys Thr
           195                 200                 205
   Ser Pro Arg Arg Leu Ala Asn Lys Arg Ile Gly Gln Ile Lys Ser Lys
       210                 215                 220
   Lys Phe Arg Cys Ser Gly Ser Ala His His His His His His
   225                 230                 235
<210> 19
   <211> 726
   <212> DNA
   <213> Homo sapiens
<220>
   <221> CDS
   <222> (1)..(726)
<400> 19
   atg tac agg atg caa ctc ctg tct tgc att gca cta agt ctt gca ctt      48
   Met Tyr Arg Met Gln Leu Leu Ser Cys Ile Ala Leu Ser Leu Ala Leu
   1               5                   10                  15
   gtc acg aat tca ctg tcc tcc ggc tcc tgc ccg gcc atg tgc acc tgc      96
   Val Thr Asn Ser Leu Ser Ser Gly Ser Cys Pro Ala Met Cys Thr Cys
               20                  25                  30
   agc aat ggc atc gtg gac tgt cgt gga aaa ggc ctc act gcc atc ccg     144
   Ser Asn Gly Ile Val Asp Cys Arg Gly Lys Gly Leu Thr Ala Ile Pro
           35                  40                  45
   gcc aac ctg ccc gag acc atg acg gag ata cgc ctg gag ctg aac ggc     192
   Ala Asn Leu Pro Glu Thr Met Thr Glu Ile Arg Leu Glu Leu Asn Gly
       50                  55                  60
   atc aag tcc atc cct cct gga gcc ttc tca ccc tac aga aag cta cgg     240
   Ile Lys Ser Ile Pro Pro Gly Ala Phe Ser Pro Tyr Arg Lys Leu Arg
   65                  70                  75                  80
   agg ata gac ctg agc aac aat cag atc gct gag att gca ccc gac gcc     288
   Arg Ile Asp Leu Ser Asn Asn Gln Ile Ala Glu Ile Ala Pro Asp Ala
                   85                  90                  95
   ttc cag ggc ctc cgc tcc ctg aac tcg ctg gtc ctc tat gga aac aag     336
   Phe Gln Gly Leu Arg Ser Leu Asn Ser Leu Val Leu Tyr Gly Asn Lys
               100                 105                 110
   atc aca gac ctc ccc cgt ggt gtg ttt gga ggc cta tac acc cta cag     384
   Ile Thr Asp Leu Pro Arg Gly Val Phe Gly Gly Leu Tyr Thr Leu Gln
           115                 120                 125
   ctc ctg ctc ctg aat gcc aac aag atc aac tgc atc cgg ccc gat gcc      432
   Leu Leu Leu Leu Asn Ala Asn Lys Ile Asn Cys Ile Arg Pro Asp Ala
       130                 135                 140
   ttc cag gac ctg cag aac ctc tca ctg ctc tcc ctg tat gac aac aag      480
   Phe Gln Asp Leu Gln Asn Leu Ser Leu Leu Ser Leu Tyr Asp Asn Lys
   145                 150                 155                 160
   atc cag agc ctc gcc aag ggc act ttc acc tcc ctg cgg gcc atc cag      528
   Ile Gln Ser Leu Ala Lys Gly Thr Phe Thr Ser Leu Arg Ala Ile Gln
                   165                 170                 175
   act ctg cac ctg gcc cag aac cct ttc att tgc gac tgt aac ctc aag      576
   Thr Leu His Leu Ala Gln Asn Pro Phe Ile Cys Asp Cys Asn Leu Lys
               180                 185                 190
   tgg ctg gca gac ttc ctg cgc acc aat ccc atc gag acg agt ggt gcc      624
   Trp Leu Ala Asp Phe Leu Arg Thr Asn Pro Ile Glu Thr Ser Gly Ala
           195                 200                 205
   cgc tgt gcc agt ccc cgg cgc ctc gcc aac aag cgc atc ggg cag atc      672
   Arg Cys Ala Ser Pro Arg Arg Leu Ala Asn Lys Arg Ile Gly Gln Ile
       210                 215                 220
   aag agc aag aag ttc cgg tgc tca ggt agc gct cat cac cat cat cat      720
   Lys Ser Lys Lys Phe Arg Cys Ser Gly Ser Ala His His His His His
   225                 230                 235                 240
   cac tga                                                              726
   His
<210> 20
   <211> 241
   <212> PRT
   <213> Homo sapiens
<400> 20
   Met Tyr Arg Met Gln Leu Leu Ser Cys Ile Ala Leu Ser Leu Ala Leu
   1               5                   10                  15
   Val Thr Asn Ser Leu Ser Ser Gly Ser Cys Pro Ala Met Cys Thr Cys
               20                  25                  30
   Ser Asn Gly Ile Val Asp Cys Arg Gly Lys Gly Leu Thr Ala Ile Pro
           35                  40                  45
   Ala Asn Leu Pro Glu Thr Met Thr Glu Ile Arg Leu Glu Leu Asn Gly
       50                  55                  60
   Ile Lys Ser Ile Pro Pro Gly Ala Phe Ser Pro Tyr Arg Lys Leu Arg
   65                  70                  75                  80
   Arg Ile Asp Leu Ser Asn Asn Gln Ile Ala Glu Ile Ala Pro Asp Ala
                   85                  90                  95
   Phe Gln Gly Leu Arg Ser Leu Asn Ser Leu Val Leu Tyr Gly Asn Lys
   100 105 110
   Ile Thr Asp Leu Pro Arg Gly Val Phe Gly Gly Leu Tyr Thr Leu Gln
   115 120 125
   Leu Leu Leu Leu Asn Ala Asn Lys Ile Asn Cys Ile Arg Pro Asp Ala
   130 135 140
   Phe Gln Asp Leu Gln Asn Leu Ser Leu Leu Ser Leu Tyr Asp Asn Lys
   145 150 155 160
   Ile Gln Ser Leu Ala Lys Gly Thr Phe Thr Ser Leu Arg Ala Ile Gln
   165 170 175
   Thr Leu His Leu Ala Gln Asn Pro Phe Ile Cys Asp Cys Asn Leu Lys
   180 185 190
   Trp Leu Ala Asp Phe Leu Arg Thr Asn Pro Ile Glu Thr Ser Gly Ala
   195 200 205
   Arg Cys Ala Ser Pro Arg Arg Leu Ala Asn Lys Arg Ile Gly Gln Ile
   210 215 220
   Lys Ser Lys Lys Phe Arg Cys Ser Gly Ser Ala His His His His His
   225 230 235 240
   His
<210> 21
   <211> 726
   <212> DNA
   <213> Homo sapiens
<220>
   <221> CDS
   <222> (1)..(726)
<400> 21
   atg tac agg atg caa ctc ctg tct tgc att gca cta agt ctt gca ctt 48
   Met Tyr Arg Met Gln Leu Leu Ser Cys Ile Ala Leu Ser Leu Ala Leu
   1 5 10 15
   gtc acg aat tca gcc aac tcc atc tcc tgc cct tcg ccc tgc acg tgc 96
   Val Thr Asn Ser Ala Asn Ser Ile Ser Cys Pro Ser Pro Cys Thr Cys
   20 25 30
   agc aat aac atc gtg gac tgt cga gga aag ggc ttg atg gag att cct 144
   Ser Asn Asn Ile Val Asp Cys Arg Gly Lys Gly Leu Met Glu Ile Pro
   35 40 45
   gcc aac ttg ccg gag ggc atc gtc gaa ata cgc cta gaa cag aac tcc       192
   Ala Asn Leu Pro Glu Gly Ile Val Glu Ile Arg Leu Glu Gln Asn Ser
       50                  55                  60
   atc aaa gcc atc cct gca gga gcc ttc acc cag tac aag aaa ctg aag       240
   Ile Lys Ala Ile Pro Ala Gly Ala Phe Thr Gln Tyr Lys Lys Leu Lys
   65                  70                  75                  80
   cga ata gac atc agc aag aat cag ata tcg gat att gct cca gat gcc       288
   Arg Ile Asp Ile Ser Lys Asn Gln Ile Ser Asp Ile Ala Pro Asp Ala
                   85                  90                  95
   ttc cag ggc ctg aaa tca ctc aca tcg ctg gtc ctg tat ggg aac aag       336
   Phe Gln Gly Leu Lys Ser Leu Thr Ser Leu Val Leu Tyr Gly Asn Lys
               100                 105                 110
   atc acc gag att gcc aag gga ctg ttt gat ggg ctg gtg tcc cta cag       384
   Ile Thr Glu Ile Ala Lys Gly Leu Phe Asp Gly Leu Val Ser Leu Gln
           115                 120                 125
   ctg ctc ctc ctc aat gcc aac aag atc aac tgc ctg cgg gtg aac acg       432
   Leu Leu Leu Leu Asn Ala Asn Lys Ile Asn Cys Leu Arg Val Asn Thr
       130                 135                 140
   ttt cag gac ctg cag aac ctc aac ttg ctc tcc ctg tat gac aac aag       480
   Phe Gln Asp Leu Gln Asn Leu Asn Leu Leu Ser Leu Tyr Asp Asn Lys
   145                 150                 155                 160
   ctg cag acc atc agc aag ggg ctc ttc gcc cct ctg cag tcc atc cag       528
   Leu Gln Thr Ile Ser Lys Gly Leu Phe Ala Pro Leu Gln Ser Ile Gln
                   165                 170                 175
   aca ctc cac tta gcc caa aac cca ttt gtg tgc gac tgc cac ttg aag       576
   Thr Leu His Leu Ala Gln Asn Pro Phe Val Cys Asp Cys His Leu Lys
               180                 185                 190
   tgg ctg gcc gac tac ctc cag gac aac ccc atc gag aca agc ggg gcc       624
   Trp Leu Ala Asp Tyr Leu Gln Asp Asn Pro Ile Glu Thr Ser Gly Ala
           195                 200                 205
   cgc tgc agc agc ccg cgc cga ctc gcc aac aag cgc atc agc cag atc       672
   Arg Cys Ser Ser Pro Arg Arg Leu Ala Asn Lys Arg Ile Ser Gln Ile
       210                 215                 220
   aag agc aag aag ttc cgg tgc tca ggt agc gct cat cac cat cat cat       720
   Lys Ser Lys Lys Phe Arg Cys Ser Gly Ser Ala His His His His His
   225                 230                 235                 240
   cac tga                                                               726
   His
<210> 22
   <211> 241
   <212> PRT
   <213> Homo sapiens
<400> 22
   Met Tyr Arg Met Gln Leu Leu Ser Cys Ile Ala Leu Ser Leu Ala Leu
   1               5                   10                  15
   Val Thr Asn Ser Ala Asn Ser Ile Ser Cys Pro Ser Pro Cys Thr Cys
               20                  25                  30
   Ser Asn Asn Ile Val Asp Cys Arg Gly Lys Gly Leu Met Glu Ile Pro
           35                  40                  45
   Ala Asn Leu Pro Glu Gly Ile Val Glu Ile Arg Leu Glu Gln Asn Ser
       50                  55                  60
   Ile Lys Ala Ile Pro Ala Gly Ala Phe Thr Gln Tyr Lys Lys Leu Lys
   65                  70                  75                  80
   Arg Ile Asp Ile Ser Lys Asn Gln Ile Ser Asp Ile Ala Pro Asp Ala
                   85                  90                  95
   Phe Gln Gly Leu Lys Ser Leu Thr Ser Leu Val Leu Tyr Gly Asn Lys
               100                 105                 110
   Ile Thr Glu Ile Ala Lys Gly Leu Phe Asp Gly Leu Val Ser Leu Gln
           115                 120                 125
   Leu Leu Leu Leu Asn Ala Asn Lys Ile Asn Cys Leu Arg Val Asn Thr
       130                 135                 140
   Phe Gln Asp Leu Gln Asn Leu Asn Leu Leu Ser Leu Tyr Asp Asn Lys
   145                 150                 155                 160
   Leu Gln Thr Ile Ser Lys Gly Leu Phe Ala Pro Leu Gln Ser Ile Gln
                   165                 170                 175
   Thr Leu His Leu Ala Gln Asn Pro Phe Val Cys Asp Cys His Leu Lys
               180                 185                 190
   Trp Leu Ala Asp Tyr Leu Gln Asp Asn Pro Ile Glu Thr Ser Gly Ala
           195                 200                 205
   Arg Cys Ser Ser Pro Arg Arg Leu Ala Asn Lys Arg Ile Ser Gln Ile
       210                 215                 220
   Lys Ser Lys Lys Phe Arg Cys Ser Gly Ser Ala His His His His His
   225                 230                 235                 240
   His
<210> 23
   <211> 1362
   <212> DNA
   <213> Homo sapiens
<220>
   <221> CDS
   <222> (1)..(1362)
<400> 23
   atg tac agg atg caa ctc ctg tct tgc att gca cta agt ctt gca ctt      48
   Met Tyr Arg Met Gln Leu Leu Ser Cys Ile Ala Leu Ser Leu Ala Leu
   1               5                   10                  15
   gtc acg aat tca ctt cgt cag gaa gat ttt cca cct cgc att gtt gaa      96
   Val Thr Asn Ser Leu Arg Gln Glu Asp Phe Pro Pro Arg Ile Val Glu
               20                  25                  30
   cac cct tca gac ctg att gtc tca aaa gga gaa cct gca act ttg aac     144
   His Pro Ser Asp Leu Ile Val Ser Lys Gly Glu Pro Ala Thr Leu Asn
           35                  40                  45
   tgc aaa gct gaa ggc cgc ccc aca ccc act att gaa tgg tac aaa ggg     192
   Cys Lys Ala Glu Gly Arg Pro Thr Pro Thr Ile Glu Trp Tyr Lys Gly
       50                  55                  60
   gga gag aga gtg gag aca gac aaa gat gac cct cgc tca cac cga atg     240
   Gly Glu Arg Val Glu Thr Asp Lys Asp Asp Pro Arg Ser His Arg Met
   65                  70                  75                  80
   ttg ctg ccg agt gga tct tta ttt ttc tta cgt ata gta cat gga cgg     288
   Leu Leu Pro Ser Gly Ser Leu Phe Phe Leu Arg Ile Val His Gly Arg
                   85                  90                  95
   aaa agt aga cct gat gaa gga gtc tat gtc tgt gta gca agg aat tac     336
   Lys Ser Arg Pro Asp Glu Gly Val Tyr Val Cys Val Ala Arg Asn Tyr
               100                 105                 110
   ctt gga gag gct gtg agc cac aat gca tcg ctg gaa gta gcc ata ctt     384
   Leu Gly Glu Ala Val Ser His Asn Ala Ser Leu Glu Val Ala Ile Leu
           115                 120                 125
   cgg gat gac ttc aga caa aac cct tcg gat gtc atg gtt gca gta gga     432
   Arg Asp Asp Phe Arg Gln Asn Pro Ser Asp Val Met Val Ala Val Gly
       130                 135                 140
   gag cct gca gta atg gaa tgc caa cct cca cga ggc cat cct gag ccc     480
   Glu Pro Ala Val Met Glu Cys Gln Pro Pro Arg Gly His Pro Glu Pro
   145                 150                 155                 160
   acc att tca tgg aag aaa gat ggc tct cca ctg gat gat aaa gat gaa     528
   Thr Ile Ser Trp Lys Lys Asp Gly Ser Pro Leu Asp Asp Lys Asp Glu
                   165                 170                 175
   aga ata act ata cga gga gga aag ctc atg atc act tac acc cgt aaa     576
   Arg Ile Thr Ile Arg Gly Gly Lys Leu Met Ile Thr Tyr Thr Arg Lys
               180                 185                 190
   agt gac gct ggc aaa tat gtt tgt gtt ggt acc aat atg gtt ggg gaa     624
   Ser Asp Ala Gly Lys Tyr Val Cys Val Gly Thr Asn Met Val Gly Glu
           195                 200                 205
   cgt gag agt gaa gta gcc gag ctg act gtc tta gag aga cca tca ttt     672
   Arg Glu Ser Glu Val Ala Glu Leu Thr Val Leu Glu Arg Pro Ser Phe
       210                 215                 220
   gtg gag tcc aag tac ggc cct cct tgc cct ccc tgc cct gcc cct gag        720
   Val Glu Ser Lys Tyr Gly Pro Pro Cys Pro Pro Cys Pro Ala Pro Glu
   225                 230                 235                 240
   ttc gag ggc gga cct agc gtg ttc ctg ttc cct cct aag cct aag gac        768
   Phe Glu Gly Gly Pro Ser Val Phe Leu Phe Pro Pro Lys Pro Lys Asp
                   245                 250                 255
   acc ctg atg atc tcc cgg acc cct gag gtg acc tgt gtg gtg gtg gac        816
   Thr Leu Met Ile Ser Arg Thr Pro Glu Val Thr Cys Val Val Val Asp
               260                 265                 270
   gtg tcc cag gag gac cct gag gtc cag ttc aac tgg tac gtg gac ggc        864
   Val Ser Gln Glu Asp Pro Glu Val Gln Phe Asn Trp Tyr Val Asp Gly
           275                 280                 285
   gtg gag gtg cac aac gcc aag acc aag cct cgg gag gag cag ttc aat        912
   Val Glu Val His Asn Ala Lys Thr Lys Pro Arg Glu Glu Gln Phe Asn
       290                 295                 300
   tcc acc tac cgg gtg gtg tct gtg ctg acc gtg ctg cac cag gac tgg        960
   Ser Thr Tyr Arg Val Val Ser Val Leu Thr Val Leu His Gln Asp Trp
   305                 310                 315                 320
   ctg aac ggc aaa gaa tac aag tgt aag gtc tcc aac aag ggc ctg ccc       1008
   Leu Asn Gly Lys Glu Tyr Lys Cys Lys Val Ser Asn Lys Gly Leu Pro
                   325                 330                 335
   tcc tcc atc gag aaa acc atc tcc aag gcc aag ggc cag cct agg gag       1056
   Ser Ser Ile Glu Lys Thr Ile Ser Lys Ala Lys Gly Gln Pro Arg Glu
               340                 345                 350
   cct cag gtg tac acc ctg cct cct agc cag gaa gag atg acc aag aac       1104
   Pro Gln Val Tyr Thr Leu Pro Pro Ser Gln Glu Glu Met Thr Lys Asn
           355                 360                 365
   cag gtg tcc ctg acc tgt ctg gtg aag ggc ttc tac cct tcc gac atc       1152
   Gln Val Ser Leu Thr Cys Leu Val Lys Gly Phe Tyr Pro Ser Asp Ile
       370                 375                 380
   gcc gtg gag tgg gag tcc aac ggc cag cct gag aac aac tac aag acc       1200
   Ala Val Glu Trp Glu Ser Asn Gly Gln Pro Glu Asn Asn Tyr Lys Thr
   385                 390                 395                 400
   acc cct cct gtg ctg gac tcc gac ggc tcc ttc ttc ctg tac tcc agg       1248
   Thr Pro Pro Val Leu Asp Ser Asp Gly Ser Phe Phe Leu Tyr Ser Arg
                   405                 410                 415
   ctg acc gtg gac aag tcc cgg tgg cag gag ggc aac gtc ttt tcc tgc       1296
   Leu Thr Val Asp Lys Ser Arg Trp Gln Glu Gly Asn Val Phe Ser Cys
               420                 425                 430
   tcc gtg atg cac gag gcc ctg cac aac cac tac acc cag aag tcc ctg       1344
   Ser Val Met His Glu Ala Leu His Asn His Tyr Thr Gln Lys Ser Leu
           435                 440                 445
   tcc ctg tct ctg ggc tga                                               1362
   Ser Leu Ser Leu Gly
       450
<210> 24
   <211> 453
   <212> PRT
   <213> Homo sapiens
<400> 24
   Met Tyr Arg Met Gln Leu Leu Ser Cys Ile Ala Leu Ser Leu Ala Leu
   1               5                   10                  15
   Val Thr Asn Ser Leu Arg Gln Glu Asp Phe Pro Pro Arg Ile Val Glu
               20                  25                  30
   His Pro Ser Asp Leu Ile Val Ser Lys Gly Glu Pro Ala Thr Leu Asn
           35                  40                  45
   Cys Lys Ala Glu Gly Arg Pro Thr Pro Thr Ile Glu Trp Tyr Lys Gly
       50                  55                  60
   Gly Glu Arg Val Glu Thr Asp Lys Asp Asp Pro Arg Ser His Arg Met
   65                  70                  75                  80
   Leu Leu Pro Ser Gly Ser Leu Phe Phe Leu Arg Ile Val His Gly Arg
                   85                  90                  95
   Lys Ser Arg Pro Asp Glu Gly Val Tyr Val Cys Val Ala Arg Asn Tyr
               100                 105                 110
   Leu Gly Glu Ala Val Ser His Asn Ala Ser Leu Glu Val Ala Ile Leu
           115                 120                 125
   Arg Asp Asp Phe Arg Gln Asn Pro Ser Asp Val Met Val Ala Val Gly
       130                 135                 140
   Glu Pro Ala Val Met Glu Cys Gln Pro Pro Arg Gly His Pro Glu Pro
   145                 150                 155                 160
   Thr Ile Ser Trp Lys Lys Asp Gly Ser Pro Leu Asp Asp Lys Asp Glu
                   165                 170                 175
   Arg Ile Thr Ile Arg Gly Gly Lys Leu Met Ile Thr Tyr Thr Arg Lys
               180                 185                 190
   Ser Asp Ala Gly Lys Tyr Val Cys Val Gly Thr Asn Met Val Gly Glu
           195                 200                 205
   Arg Glu Ser Glu Val Ala Glu Leu Thr Val Leu Glu Arg Pro Ser Phe
       210                 215                 220
   Val Glu Ser Lys Tyr Gly Pro Pro Cys Pro Pro Cys Pro Ala Pro Glu
   225                 230                 235                 240
   Phe Glu Gly Gly Pro Ser Val Phe Leu Phe Pro Pro Lys Pro Lys Asp
                   245                 250                 255
   Thr Leu Met Ile Ser Arg Thr Pro Glu Val Thr Cys Val Val Val Asp
               260                 265                 270
   Val Ser Gln Glu Asp Pro Glu Val Gln Phe Asn Trp Tyr Val Asp Gly
           275                 280                 285
   Val Glu Val His Asn Ala Lys Thr Lys Pro Arg Glu Glu Gln Phe Asn
       290                 295                 300
   Ser Thr Tyr Arg Val Val Ser Val Leu Thr Val Leu His Gln Asp Trp
   305                 310                 315                 320
   Leu Asn Gly Lys Glu Tyr Lys Cys Lys Val Ser Asn Lys Gly Leu Pro
                   325                 330                 335
   Ser Ser Ile Glu Lys Thr Ile Ser Lys Ala Lys Gly Gln Pro Arg Glu
               340                 345                 350
   Pro Gln Val Tyr Thr Leu Pro Pro Ser Gln Glu Glu Met Thr Lys Asn
           355                 360                 365
   Gln Val Ser Leu Thr Cys Leu Val Lys Gly Phe Tyr Pro Ser Asp Ile
       370                 375                 380
   Ala Val Glu Trp Glu Ser Asn Gly Gln Pro Glu Asn Asn Tyr Lys Thr
   385                 390                 395                 400
   Thr Pro Pro Val Leu Asp Ser Asp Gly Ser Phe Phe Leu Tyr Ser Arg
                   405                 410                 415
   Leu Thr Val Asp Lys Ser Arg Trp Gln Glu Gly Asn Val Phe Ser Cys
               420                 425                 430
   Ser Val Met His Glu Ala Leu His Asn His Tyr Thr Gln Lys Ser Leu
           435                 440                 445
   Ser Leu Ser Leu Gly
       450

## Description

The present application describes a recombinant protein Robo1-Fc and the use thereof for treating diseases in which a Slit protein is overexpressed. In particular, the present application describes the use of a Robo-Fc protein for treating cancer, especially hepatocarcinomas. It also describes a composition comprising such a recombinant protein. Another aspect of the application describes the use of a Robo1-Fc molecule as a diagnostic tool for detecting the overexpression of a molecule of the Slit family in a patient.

Slit ligands were first of all described for their role in repelling axonal growth in neuronal development. Since then, the Robo/Slit regulatory pathway has also been described in tumour angiogenesis. Specifically, the Robo4/Slit2 pathway has been described for inhibiting the response of endothelial cells to VEGF (Jones C.A. et al. Nat. Med 14, 448-453 (2008)). Regulation by the Robo/Slit pathway makes it possible to channel the excessive proliferation of endothelial non-mature neovessels or buds (non-productive angiogenesis) and to mature these vessels. The expression of Slit2 at the transcriptional level has been demonstrated in several human cancer lines, in particular HCT116 derived from colon carcinoma, Skov-3 derived from ovarian carcinoma, HeLa derived from cervical cancer, MDA-MB-435 derived from melanoma, Hec-1A derived from uterine cancer and, finally, 769-P derived from renal carcinoma (Stella MC et al., Mol Biol Cell 2009 Vol. 20, Issue 2, 642-657). Overexpression of the Slit2 protein has also been demonstrated on human tissues derived from carcinomas: oral carcinoma (Wang et al. 2008, Cancer Sci. 2008 Mar; 99(3): 510-7), prostate carcinoma (Latil et al. 2003 Int J Cancer. 2003 Jan 20; 103(3): 306-15), colon carcinoma (Wang et al. 2003, Cancer Cell, Volume 4, Issue 1, July 2003, Pages 19-29), and liver carcinoma (Avci et al, 2008, BMC cancer, 8: 392). More recently, overexpression of the Slit2 protein has been shown on samples of endometriosis (Shen et al, 2009, AJP 175 (2): 479).

The Robo1 protein exists as two isoforms a and b. The extracelllar domain of the Robo1 protein isoform b (NP_598334) comprises 5 immunoglobulin domains: Ig1, Ig2, Ig3, Ig4 and Ig5. The Robo protein interacts with the Slit ligands at the level of the Ig1 and Ig2 domains. Liu et al. (2004, Mol. Cell Neurosci, 26: 232-240) have demonstrated the importance of the Ig1 and Ig2 domains in the interaction with Slit and in the activity of Robo (chemorepulsion). The European application EP 1744462 describes that Robo1 protein is highly expressed in cancer cells and that it can thus be used as a marker of cancer.

The use of antibodies specific for the Robo1 and the Slit2 proteins has been described in application WO2003/075860. These antibodies make it possible to inhibit tumour angiogenesis. The use of antibodies specific to Robo and Slit proteins, fragments of Robo protein or fusion proteins comprising a fragment of the Robo protein have also been described in application WO2011/134420 to treat or control lymph vessel formation.

However, it would be advantageous to propose an alternative approach for treating cancer, in particular a molecule capable of inhibiting the Slit2 signalling pathway.

The inventors have developed a strategy of soluble chimeric Robo1 receptors capable of binding Slit ligands and consequently, of inhibiting the intracellular signalling of the Robo/Slit pathway. Surprisingly, the Robo1-Fc molecules according to the invention have an anti-angiogenesis effect by preventing the formation of mature vessels and not by inhibiting the proliferation of endothelial cells.

The inventors have shown that the Robo1-Fc molecules also have an antitumour effect in lung and liver cancer.

### DETAILED DESCRIPTION OF THE INVENTION

The present application discloses a Robo-Fc recombinant protein comprising the extracellular domain of the Robo1 protein, isoform b or a part of this domain, a junction region (linker) and an immunoglobulin Fc domain.

The present invention concerns a recombinant protein comprising the extracellular domain derived from Robo1 protein or a part of this domain, a linker and an immunoglobulin Fc domain, for use in the treatment of hepatocarcinoma.

In one particular embodiment, the extracellular domain of the Robo1 protein, isoform b consists of the Ig1 and Ig2 domains. These domains correspond to the peptide of SEQ ID NO.2 encoded by the nucleotide sequence SEQ ID NO.1, or a sequence having at least 80%, 85%, 90%, 95% or 99% identity with the sequence SEQ ID NO.2.

The fusion protein also comprises a junction region, also called "linker". In the context of the present invention, the linker makes it possible to give the recombinant protein stability, in particular by limiting *in vivo* cleavage. Linkers that can be used in a Robo1-Fc molecule are, for example, GluArgProSerPheVal and GlyGlyGlyGlySer. Those skilled in the art have sufficient knowledge to select a linker suitable for this use.

The Fc domain of the Robo1-Fc recombinant molecules according to the invention corresponds to a crystallizable fragment of an immunoglobulin. This Fc fragment can come from various immunoglobulins IgG1, IgG2, IgG3 or IgG4. It is responsible for the effector function of the immune response (WO2008/065543).

In one embodiment according to the invention, the Fc domain comes from an IgG4 immunoglobulin. In one particular embodiment, at least one point mutation or deletion has been introduced into the IgG4 Fc domain so as to increase the stability of the molecule, in particular by stabilizing the hinge region made up of the two Fc domains (Angla et al., 1993, Mol. Immunol., 30: 105-108), to reduce or eliminate the residual activity of the IgG4-Fc, in particular the effector activity (WO 97/09351), and to increase the homogeneity during the production of the recombinant protein. In particular, at least two point mutations, preferably three point mutations, have been introduced into the IgG4 Fc domain. In the Robo1-Fc L1, Robo1-Fc L2 and Robo1-Fc L3 molecules according to the invention, the preferred mutations are the following:
- S241 P (Kabat numbering) in order to stabilize the disulphide-bridge bonding in the Fc hinge region;
- L248E (Kabat numbering) in order to eliminate the residual effector activity of the IgG4-Fc domain;
- absence of the C-terminal lysine in order to reduce the heterogeneity of the protein.

Robo1-Fc molecules according to the invention comprising the Ig1 and Ig2 domains of Robo1 isoform b, a linker and a mutated IgG4 domain as described above are Robo1-Fc L1, Robo1-Fc L2 and Robo1-Fc L3.

Robo1-Fc L1 corresponds to the protein of sequence SEQ ID NO.4, encoded by the nucleotide sequence SEQ ID NO.3.

Robo1-Fc L2 corresponds to the protein of sequence SEQ ID NO.6, encoded by the nucleotide sequence SEQ ID NO.5.

Robo1-Fc L1 and Robo1-Fc L2 differ by virtue of the nature of the linker.

Robo1-Fc L3 corresponds to the protein of sequence SEQ ID NO.24, encoded by the nucleotide sequence SEQ ID NO.23.

In one particular embodiment of the invention, one or more point mutations or deletions have been introduced in order to increase the homogeneity during production. In particular, one amino acid, preferably two amino acids, have been truncated in the N-terminal position. Such a Robo-1-Fc molecule according to the invention is the Robo1-Fc L3 molecule, in which the two amino acids (Ser20 and Arg21) have been truncated and in which the amino acid Leu 22 has been fused to the signal peptide of interleukin 2.

The homogeneity of the Robo1-Fc molecules according to the invention is also increased by deleting the C-terminal Lys as previously described.

The application also describes proteins of which the protein sequence corresponds to the sequences SEQ ID NO.2, SEQ ID NO.4, SEQ ID NO.6 or SEQ ID NO.24 or has at least 80%, 85%, 90%, 95% or 99% identity with the sequences SEQ ID NO.2, SEQ ID NO.4, SEQ ID NO.6 or SEQ ID NO.24. These protein variants have the same biological activity as the proteins having the sequence SEQ ID NO.2, SEQ ID NO.4, SEQ ID NO.6 or SEQ ID NO.24, in particular their ability to interact with the proteins of the Slit family.

The Robo1-Fc proteins according to the invention can be produced by transfection of expression plasmids encoding these proteins into any cell type suitable for the expression of eukaryotic recombinant proteins: HEK293, CHO, etc., and then recovered in the culture supernatant and purified according to conventional methods.

Characterization of the Robo1-Fc L1, Robo1-Fc L2 and Robo1-Fc L3 proteins according to the invention has made it possible to confirm that they have the qualities required for their administration as a biotherapeutic agent.

A Robo1-Fc protein according to the invention has the ability to interact with a protein of the Slit family.

The Robo1-Fc proteins according to the invention specifically recognize the human Slit1, Slit2 and Slit3 proteins and the murine Slit2 protein, in particular by interaction with their D2 domain. Their affinity is similar with respect to the 3 members of the Slit family.

The application also describes the nucleic acid molecules encoding the proteins according to the invention.

Thus, the nucleic acid molecules corresponding to the sequences SEQ ID NO.1, SEQ ID NO.3, SEQ ID NO.5 or SEQ ID NO.23 or having at least 80%, 85%, 90%, 95% or 99% identity with the molecules having the sequence SEQ ID NO.1, SEQ ID NO.3, SEQ ID NO.5 or SEQ ID NO.23 are described herein.

The application also discloses the use of a Robo1-Fc protein according to the invention for treating diseases in which a protein of the Slit family is overexpressed.

The proteins of the Slit family which can be targeted by the Robo1-Fcs described herein are Slit1, Slit2 or Slit3.

It has been shown that Robo1 can interact with the various members of the Slit family. Consequently, it is advantageous to note that the Robo1-Fc proteins described herein are capable of simultaneously inhibiting the signalling pathways mediated by Slit1, Slit2 and Slit3, which makes it possible to broaden the therapeutic spectrum in comparison with the antibodies which are specific for only one of these pathways.

As described herein, a Robo1-Fc protein may be used for treating diseases in which a protein of the Slit family is overexpressed, by inhibiting angiogenesis without inhibiting endothelial cell proliferation. This anti-angiogenic activity linked to a vessel maturation defect is called "non-productive angiogenesis".

Specifically, the experimental studies have shown that the Robo1-Fc molecules according to the invention are capable of inhibiting the formation of tubules, without inhibiting endothelial cell proliferation. They make it possible to very significantly reduce the tumour volume in a murine model of lung cancer.

As described herein, the Robo1-Fc proteins that can be used for treating diseases in which a Slit protein is overexpressed are Robo1-Fc L1, Robo1-Fc L2 and Robo1-Fc L3, and the molecules which are derived therefrom, include, in particular, point mutations or deletions aimed at increasing the homogeneity during production without significantly modifying the biological properties of these molecules.

In general, the diseases that can be treated with a Robo-1-Fc protein described herein are all the diseases in which inhibition of the Slit pathway can have a therapeutic effect, in particular the diseases in which a protein of the Slit family is overexpressed, and in particular those in which Slit2 is overexpressed.

Since the Robo1-Fc molecules described herein specifically bind the Slit2 molecule, it is interesting to note that said molecules are capable of simultaneously inhibiting the two pathways in which Slit2 is involved, namely the Robo1/Slit2 and Robo4/Slit2 pathways.

As described herein, the Robo1-Fc proteins described herein may be used for treating cancer, in particular pancreatic cancer, colon cancer, colorectal cancer with or without lymphatic metastasis, breast cancer, lung cancer and lung metastases, ovarian cancer, cervical cancer, melanomas, renal cancer, oral cancer, prostate cancer, liver cancer, etc. In a specific embodiment, said cancer is a cancer in which Slit2 is overexpressed.

The Robo-Fc proteins described herein may be used for treating lung cancer.

As described herein, the Robo-Fc proteins may be used for treating liver cancer, such as e.g. hepatocarcinoma, hepatocellular carcinoma (HCC), fibrolamellar carcinoma (a pathological variant of hepatocellular carcinoma), cholangiocarcinoma, hepatoblastoma, or angiosarcoma of the liver.

The Robo-Fc proteins according to the invention are used for treating hepatocarcinoma.

The Robo1-Fc molecules described herein can also be used as an anticancer medicament as an alternative to or as a supplement to the current therapies.

In particular, these molecules can be administered in combination (optionally simultaneously) with anticancer compounds.

The application describes a composition comprising a Robo1-Fc protein as defined above and one or more pharmaceutically acceptable excipients.

The Robo1-Fc proteins described herein can be formulated in pharmaceutical compositions with a view to topical, oral, parenteral, intranasal, intravenous, intramuscular, subcutaneous, intraocular, etc., administration. Preferably, the pharmaceutical compositions contain pharmaceutically acceptable vehicles for an injectable formulation. They may in particular be isotonic sterile saline solutions (monosodium or disodium phosphate, sodium, potassium, calcium or magnesium chloride, etc., or mixtures of such salts), or dry, in particular freeze-dried, compositions which, by addition, as appropriate, of sterilized water or of physiological saline, allow the formation of injectable solutes.

The application also discloses the use of a Robo1-Fc molecule as a diagnostic tool for detecting the overexpression of a molecule of the Slit family in a patient. This is because it has been shown that the Slit pathway is implicated in many cancers. The provision of a test for evaluating a disturbance of the Slit signalling pathway is very useful for the purpose of selecting patients capable of responding to a treatment based on the administration of a Robo1-Fc molecule.

This diagnostic tool may be in the form of a ready-to-use kit, comprising a Robo1-Fc molecule in a form suitable for it to be brought into contact with a biological sample from a patient (blood, urine, tumour biopsy) liable to exhibit an overexpression of a Slit molecule. The Robo-Fc molecule can optionally be prelabelled, and the combination of Robo-Fc and Slit is detected so as to evaluate an increase in the expression of a Slit protein in the biological sample in comparison with a control sample. This kit can, for example, be in the form of an ELISA kit.

### DESCRIPTION OF THE FIGURES

**Figure 1****:** Expression plasmids for the Robo1-Fc L1, Robo1-Fc L2 and Robo1-Fc L3 proteins.
**Figure 2****:** Evaluation of the interaction of the Robo1-Fc fusion protein variants with the Slit2 protein by ELISA.
**Figure 3****:** Affinity of Robo1-Fc for HEK293 cells not expressing Slit2, measured by FACS.
**Figure 4****:** Affinity of Robo1-Fc for HEK293 cells expressing Slit2, measured by FACS.
**Figure 5****:** Pharmacokinetic profile of the Robo1-Fc proteins after an iv injection in mice. **A**. Administration of Robo1-Fc L1, **B.** Administration of Robo1-Fc L2.
**Figure 6****:** Effect of the Robo1-Fc L1 molecule in a coculture test with endothelial cells and mesenchymal cells. **A.** Robo1-Fc L1 inhibits tubule formation in culture. **B.** Robo1-Fc L1 significantly inhibits VEGF-induced pseudotubule formation.
**Figure 7****:** Evaluation of the effect of the Robo1-Fc L1 molecule on an *ex vivo* aortic ring model in mice. **A:** Description of the protocol for preparing an aortic ring and for measuring tubules. **B:** a. Control; b. Robo1-Fc Slit2-minus 500 µg/mL + VEGF 10 ng/mL; c. Robo1-Fc L1 500 µg/mL + VEGF 10 ng/mL.
**Figure 8****:** Mean tumour weight (HepG2 cells in the left lobe of the liver of SCID mice) 28 days after injection of the cells, in the control group and in the group having received 4 injections of Robo1-Fc at 25 mg/kg.
**Figure 9****:** Mean tumour weight (Hep3B cells one lobe of the liver of SCID mice) 28 days after injection of the cells, in the control group and in the group having received 5 mg/kg of Robo1-Fc twice a week.
**Figure 10****:** Plasmatic AFP concentration in the control group and in the group having received 5 mg/kg of Robo1-Fc twice a week.

### EXAMPLES

### Example 1: Preparation of the Robo1-Fc proteins

### a. Constructs allowing the expression of the Robo1-Fc recombinant proteins used as biotherapeutic agents.

The Robo1-Fc recombinant proteins consist of a fusion between the first two immunoglobulin domains (Ig1-Ig2) of the human Robo1 protein, isoform b (NP_598334) and the Fc domain of human immunoglobulin G4 (hIgG4-Fc, SwissProt IGHG4_HUMAN).

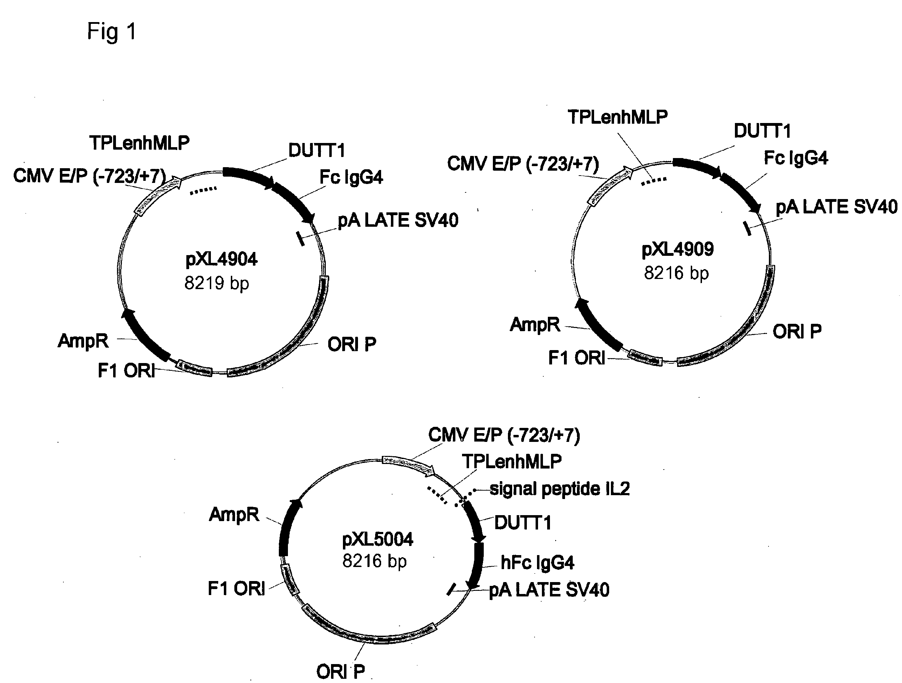

In order to obtain the Robo1-Fc L1 construct, a fragment of the cDNA (SEQ ID NO.1) corresponding to the immunoglobulin (Ig) domains Ig1 and Ig2 of this protein (SEQ ID NO.2) followed by a GluArgProSerPheVal linker was amplified by PCR using the human foetal heart cDNA library (ref. HL5042T, Clontech). This amplified fragment was then cloned into the eukaryotic expression vector pXL4904 (described in Figure 1) such that the two Ig domains of Robo1 are expressed as a fusion with the Fc domain of human IgG4 in the C-terminal position. Three point mutations were introduced into the IgG4-Fc domain in order to obtain the following characteristics: S241P (Kabat numbering) in order to stabilize the disulphide-bridge bonding in the Fc hinge region; L248E in order to eliminate the residual effector activity of the IgG4-Fc domain; absence of the C-terminal lysine in order to reduce the heterogeneity of the protein. The cDNA sequence used to express this recombinant protein corresponds to the sequence SEQ ID NO.3. The recombinant protein obtained is called Robo1-Fc L1 and corresponds to the protein sequence SEQ ID NO.4.

In order to obtain the Robo1-Fc L2 construct, the same cDNA corresponding to the Ig1-Ig2 domains but without the linker mentioned was cloned into the eukaryotic expression vector pXL4909 (described in Figure 1), which allows the expression of these Ig domains as a fusion with the same Fc domain of IgG4 containing the 3 point mutations described in the construction of Robo1-Fc L1, but this time introducing a GlyGlyGlyGlySer linker upstream of the Fc domain. The cDNA sequence used to express this recombinant protein corresponds to the sequence SEQ ID NO.5. The recombinant protein obtained is called Robo1-Fc L2 and corresponds to the protein sequence SEQ ID NO.6.

### b. Construction of the Robo1-Fc proteins used as controls

In order to obtain the Robo1-Fc Slit2-minus construct, the cDNA previously cloned into the pXL4904 plasmid was modified by PCR so as to introduce the point mutations allowing the substitutions of the leucine at position 38 to glutamine and the phenylalanine at position 89 to tyrosine. The cDNA sequence used to express this recombinant protein corresponds to the sequence SEQ ID NO.7. The recombinant protein obtained is called Robo1-Fc Slit2-minus and corresponds to the protein sequence SEQ ID NO.8.

In order to obtain the Robo1-Fc heparin-minus construct, the cDNA cloned into the pXL4904 plasmid was modified by PCR so as to introduce the point mutations allowing the substitutions of the arginine at position 97 to aniline and the lysine at position 98 to alanine. The cDNA sequence used to express this recombinant protein corresponds to the sequence SEQ ID NO.9. The recombinant protein obtained is called Robo1-Fc heparin-minus and corresponds to the protein sequence SEQ ID NO. 10.

### c. Production of the various Robo1-Fc proteins

The two proteins Robo1-Fc L1 and Robo1-Fc L2 were produced by transient transfection in the HEK293 line (FreeStyle 293-F cells ref 51-0029, Invitrogen, according to the supplier's recommendations) using the pXL4904 and pXL4909 plasmids, respectively, and the helper plasmids pXL4544 and pXL4551 allowing the expression of two N-glycan glycosylation enzymes, i.e. α-2,3-sialyltransferase and β-1,4-galactosyltransferase, as has been described in application WO2008/065543. These proteins were also produced by transfection in the CHO line (CHO-S cells, ref 11619-012, Invitrogen, according to the supplier's recommendations) using the pXL4904 and pXL4909 plasmids, respectively.

The Robo1-Fc L1 and Robo1-Fc L2 proteins expressed in the culture natant of the HEK293 cells were purified by chromatography on a protein A affinity column (MabSelect ref. 17-5199-02, Amersham Biosciences) and elution in 20 mM NaCl / 100 mM acetic acid buffer, and then formulated in PBS buffer (ref. 14190-094, Invitrogen).

The Robo1-Fc Slit2-minus and Robo1-Fc heparin-minus recombinant proteins were produced and purified in the same way.

### d. Physicochemical characterization of the Robo1-Fc recombinant proteins

SDS-PAGE and gel permeation analysis made it possible to show that the proteins were dimeric and more than 96% pure. Mass spectrometry analysis made it possible to demonstrate the identity of these proteins, the measured weight of the deglycosylated protein being in perfect agreement with the weight calculated *in silico.* Analysis of the monosaccharide composition and quantification of the N-glycan sialic acids as described by Saddic et al. 2002. (Methods Mol. Biol. 194: 23-36 and Anumula et al. 1998. Glycobiology 8: 685-694) made it possible to demonstrate that the proteins were sialylated to a very great extent. The results are given in table 1. It will be noted that the N-terminal analysis of the Robo1-Fc L1 and Robo1-Fc L2 molecules showed that these purified proteins contained a variable proportion (0 to 40%) of a form with the first 2 residues (Ser20 and Arg21) truncated

**Table 1: Robo1-Fc protein identity**

| | **Robo1-Fc L1** | **Robo1-Fc L2** |
|---|---|---|
| Weight (SDS-PAGE reducing conditions) | 56 kDa | 55 kDa |
| Weight (SEC, HPLC) | 180 kDa | 180 kDa |
| Weight (LC/MS after deglycosylation) | 48812 Da | 48410 Da |
| N-Glycan profile (HPLC) | highly sialylated 21.4% asialoglycans | highly sialylated 20.1% asialoglycans |
| Monosaccharide composition (HPLC) | complex N-glycans entirely fucosylated no O-glycans | complex N-glycans entirely fucosylated no O-glycans |

### Example 2: Preparation of the Slit proteins used as ligand

The cDNA encoding the human Slit2 protein corresponds to the reference protein NP_004778. Fragments of this cDNA were amplified by PCR using the human brain cDNA library (ref. 639300, Clontech).

The cDNA corresponding to the D2 domain was cloned into the eurkaryotic expression vector pXL4911 in order to express this domain containing a His tag in the C-terminal position. The cDNA sequence used to express this recombinant protein corresponds to the sequence SEQ ID NO.11. The recombinant protein obtained is called Slit2-D2 and corresponds to the protein sequence SEQ ID NO.12.

The cDNA corresponding to the D1-D2 domains was cloned into the eukaryotic expression vector pXL4912 in order to express these two domains containing a His tag in the C-terminal position. The cDNA sequence used to express this recombinant protein corresponds to the sequence SEQ ID NO.13. The recombinant protein obtained is called Slit2-D1D2 and corresponds to the protein sequence SEQ ID NO.14.

The cDNA corresponding to the extracellular part (Nt) of the Slit2 protein was cloned into the eukaryotic expression vector pXL5033 in order to express this protein with a His tag in the C-terminal position. The cDNA sequence used to express this recombinant protein corresponds to the sequence SEQ ID NO.15. The recombinant protein obtained is called Slit2-N and corresponds to the protein sequence SEQ ID NO.16.

The cDNA encoding the D2 domain of the murine Slit2 protein, and corresponding to the D2 domain of the described reference protein NP_848919, was obtained from the cDNA cloned into the pXL4911 plasmid. Four fragments making it possible to generate the five point mutations were generated by PCR with pXL4911 as template, and then these fragments were used as template to amplify the cDNA encoding the complete D2 domain by sequential PCR. The protein carries the Thr311Ser, Lys313Arg, Ile329Leu, Ile411Val and Pro418Ala mutations allowing it to be identical to the D2 domain of the murine Slit2 protein. This plasmid makes it possible to express the D2 domain of the murine Slit2 protein with a His tag in the C-terminal position. The cDNA sequence used to express this recombinant protein corresponds to the sequence SEQ ID NO.17. The recombinant protein obtained is called mSlit2-D2 and corresponds to the protein sequence SEQ ID NO.18.

The cDNA encoding the human Slit1 protein corresponds to the described reference protein NP_003052. A fragment of this cDNA was amplified by PCR using the human brain cDNA library (ref. 639300, Clontech). The cDNA corresponding to the D2 domain was cloned into the eukaryotic expression vector pXL5020 in order to express this domain containing a His tag in the C-terminal position. The cDNA sequence used to express this recombinant protein corresponds to the sequence SEQ ID NO.19. The recombinant protein obtained is called Slit1-D2 and corresponds to the protein sequence SEQ ID NO.20.

The cDNA encoding the human Slit3 protein corresponds to the described reference protein NP_003053. A fragment of this cDNA was amplified by PCR using the human brain cDNA library (ref. 639300, Clontech). The cDNA corresponding to the D2 domain was cloned into the eukaryotic expression vector pXL5021 in order to express this domain containing a His tag in the C-terminal position. The cDNA sequence used to express this recombinant protein corresponds to the sequence SEQ ID NO.21. The recombinant protein obtained is called Slit3-D2 and corresponds to the protein sequence SEQ ID NO.22.

The three proteins called Slit2-D2, SIit2-D1D2 and Slit2-N were produced by transient transfection in the HEK293 line (FreeStyle 293-F cells according to the supplier's recommendations, Invitrogen) using the pXL4911 plasmid (respectively, pXL4912 or pXL5033).

The Slit2-D2 and Slit2-D1D2 proteins expressed in the culture supernatant of the HEK293 cells were purified by chromatography on an Ni-chelating sepharose column (ref. 17-0409-03, Amersham Biosciences) and elution in imidazole buffer and then formulated in PBS buffer (ref. 14190-094, Invitrogen) adjusted to 1M NaCl. Mass spectrometry analysis (LC/MS) made it possible to demonstrate the identity of these proteins.

The three proteins called mSlit2-D2, Slit1-D2 and Slit3-D2 were produced, purified and characterized in a comparable manner.

### Example 3: Study of the affinity of the Robo1-Fc recombinant proteins for the Slit proteins and for heparin by means of three methods: ELISA, SPR and FACS

### a. Affinity of the Robo1-Fc proteins for heparin

In order to determine the affinity of the Robo1-Fc constructs for heparin, 2 mg of Robo1-Fc protein, purified and formulated in 10 mM phosphate, pH 7.0, were chromatographed on a heparin column (1 mL HiTrap Heparin-Sepharose HP, GE Heathcare) by elution with a linear gradient of NaCl of from 0 to 1.5 M.

Table 2 indicates the NaCl concentration of 448 mM necessary for eluting the Robo1-Fc L1 protein as described in the literature (Fukuhara, N. et al. 2008 J. Biol. Chem. 283 p16226-16234).

**Table 2: Affinity of Robo1-Fc for heparin**

| **Robo1-Fc construct** | **Detachment from the heparin column [NaCl], mM** |
|---|---|
| Robo1-Fc L1 | 448 |
| Robo1-Fc Heparin-minus | No binding |

These results show that the Robo1-Fc heparin-minus protein is not retained on this column, and therefore that it no longer has any affinity for heparin. This protein is therefore a heparin-negative mutant.

### b. Evaluation of the interaction of the Robo1-Fc protein variants with the D2 domain of the human Slit2 protein

This example describes the interaction of the two variants called Robo1-Fc L1 and Robo1-Fc L2 with their natural ligand (in these experiments, the D2 domain of human Slit2) by ELISA assay.

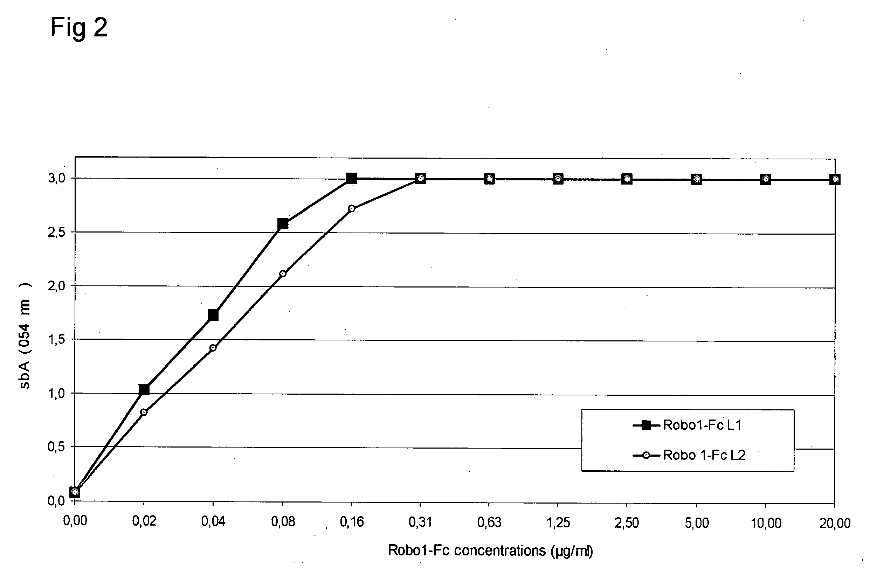

The human Slit2-D2 protein was bound to Immulon-4 enzyme-linked plates (VWR Scientific Inc. Swedesboro, NJ). A concentration range (from 20 µg/mL to 0.02 µg/mL) of the Robo1-Fc L1 and Robo1-Fc L2 variants was added and then detected by means of the peroxidase-conjugated goat anti-human IgG antibody (Sigma; ref. A0170, dilution to 1:50 000). Visualization was carried out with the TMB-H₂O₂ substrate (Interchim; ref UP664780) and the measurements were carried out with a plate reader at 450 nm. The results are reported in Figure 2. They show that the two variants Robo1-Fc L1 and L2 specifically interact with the human Slit2 protein (in particular with the D2 domain).

### c. Evaluation of the interaction of the Robo1-Fc protein with the human variants of the Slit family, namely Slit1 and Slit3

This example describes the interaction of the Robo1-Fc L1 fusion protein with the Slit1-D2, Slit2-D2 and Slit3-D2 variants by ELISA assay.

The D2 domain of the human Slit variants was bound to Immulon-4 enzyme-linked plates (VWR Scientific Inc. Swedesboro, NJ). A concentration range (from 1 µg/mL to 0.001 µg/mL) of the Robo1-Fc L1 fusion protein was added and then detected using the peroxidase-conjugated goat anti-human IgG antibody (Sigma; ref. A0170, dilution to 1:50 000). Visualization was carried out with the TMB-H₂O₂ substrate (Interchim; ref UP664780) and the measurements were carried out with a plate reader at 450 nm. Similarly, the Robo1-Fc Slit2-minus variant, which is mutated at the level of the Slit2-binding site, was evaluated according to a concentration range (from 20 µg/mL to 0.02 µg/mL) by ELISA assay under the same conditions described above. The results are reported in table 3 below.

**Table 3: Affinity of Robo1-Fc for the human variants of the Slit protein**

| **Slit protein (ligand)** | **Robo1-Fc protein** | **EC50 (µg/mL)** | **CV (%)** |
|---|---|---|---|
| Slit2-D2 | Robo1-Fc L1 | 1.32 E-01 | 3.3 |
| Slit1-D2 | Robo1-Fc L1 | 7.88 E-02 | 3.9 |
| Slit3-D2 | Robo1-Fc L1 | 2.60 E-01 | 12 |
| Slit2-D2 | Robo1-Fc Slit2-minus | 1.78 E+01 | 18 |

These results show that the Robo1-Fc protein interacts specifically with the three proteins of the family, Slit1, Slit2 and Slit3 (in particular with their D2 domain).

In addition, the Robo1-Fc Slit2-minus protein no longer has affinity for heparin and it is therefore a heparin-negative mutant.

### d. Evaluation of the interaction of the Robo1-Fc protein with the murine Slit2 protein

This example describes the interaction of the Robo1-Fc L1 fusion protein with the murine protein mSlit2-D2 by ELISA assay.

The murine protein mSlit2-D2 was bound to an Immulon-4 enzyme-linked plate (VWR Scientific Inc. Swedesboro, NJ). A concentration range (from 2 µg/mL to 0.002 µg/mL) of the Robo1-Fc L1 fusion protein was added and then detected using the peroxidase-conjugated goat anti-human IgG antibody (Sigma; ref. A0170, dilution to 1:50 000). Visualization was carried out with the TMB-H₂O₂ substrate (Interchim; ref UP664780) and the measurements were carried out with a plate reader at 450 nm. The results are reported in table 4 below.

**Table 4 : Affinity of Robo1-Fc L1 for the murine Slit2 protein**

| **Protein studied** | **EC50 (µg/mL)** | **CV (%)** |
|---|---|---|
| mSlit2-D2 | 2.21 E-01 | 18 |

These results show that the Robo1-Fc protein interacts specifically with the murine Slit2 protein

### e. Affinity of Robo1-Fc for the Slit protein measured by SPR

This example describes the determination of the affinity constant of the Robo1-Fc L1 fusion protein with the human Slit2 protein (in this experiment, Slit2-D2) by SPR (Surface Plasmon Resonance; BIAcore 2000). The interaction between the Robo1-Fc protein and the human Slit2 protein was analysed after having bound the Robo1-Fc fusion protein to a CM5 chip. The kinetic measurements were carried out according to the protocol of Canziani et al. (2004. Anal. Biochem. 325: 301-307).

**Table 5: Affinity constant of Robo-Fc L1 with human Slit2-D2 by SPR (steady-state analysis)**

| **Protein** | **K_{D} (nM)** |
|---|---|
| Robo1-Fc L1 | 32 |

A second method, which consists in determining the affinity constant between the Robo1-Fc L1 fusion protein and the human Slit2 protein, was analysed after having bound the Slit2-D2 protein to the CM5 chip. The kinetic measurements are carried out according to the protocol of Canziani et al. (2004. Anal. Biochem. 325: 301-307) using the Scatchard method according to a model with two non-equivalent binding sites.

**Table 6: Affinity constant of Robo1-Fc with human Slit2-D2 by SPR according to the two-phase Scatchard model**

| **Robo1-Fc L1** | **K_{D} (nM)** |
|---|---|
| High-affinity site | K_{D1}: 1.5 |
| Low-affinity site | K_{D2}: 160 |

### f. Affinity of Robo1-Fc for Slit measured by FACS

This example describes the affinity of the Robo1-Fc protein on HEK293 mammalian cells expressing Slit2.

The HEK293 cells described and used as in example 1 were transfected either with a ballast plasmid, having no cDNA that encodes in the mammalian cell, or the pXL4911 plasmid encoding the Slit2-D2 protein, or the pXL4912 plasmid encoding the Slit2-D1D2 protein, or pXL5033 encoding the Slit2-N protein described in example 2. The cells were distributed into 96-well plates 48 hours post-transfection, and the Robo1-Fc protein was added in a concentration range of from 0.01 to 3□mg/L for 30 min at 4°C. The Robo1-Fc protein is either the Robo1-Fc L1 biotherapeutic agent, or the Robo1-Fc Slit2-minus mutant, or the Robo1-Fc heparin-minus mutant. The cells were washed and the anti-human Fc antibody labelled with Alexa 488 (ref: A-11013, Invitrogen) was incubated for 30 min at 4°C. The labelled HEK293 cells are then quantified by FACS (Geomean).

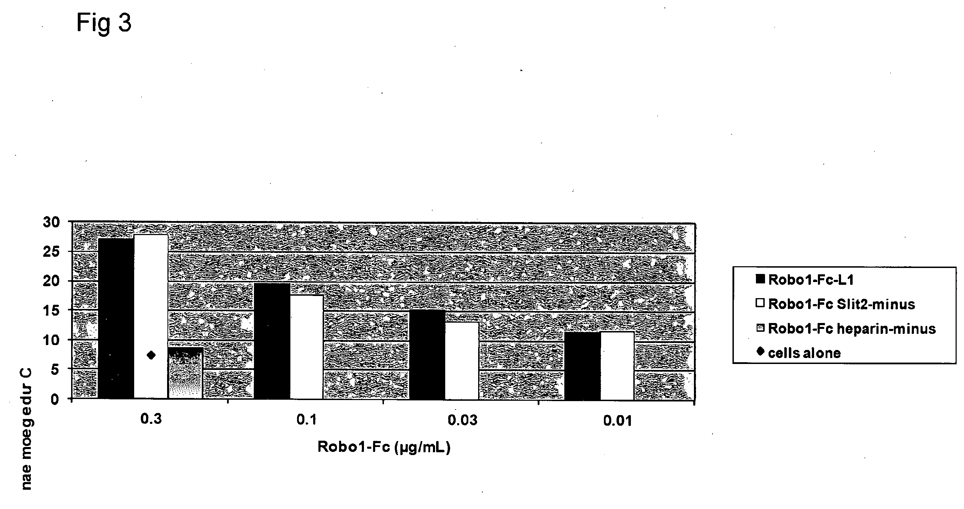

Figure 3 describes the binding of the HEK293 cells to the Robo1-Fc protein, via the fluorescence measured by the FACS in the absence of Slit2 expression. The Robo1-Fc protein and also the Robo1-Fc Slit2-minus mutant bind to the HEK293 cells, whereas the Robo1-Fc heparin-minus mutant does not bind. Robo1-Fc therefore binds partly to the HEK293 cells via heparin binding at the low Robo1-Fc concentrations of 0.3 to 0.03 mg/L.

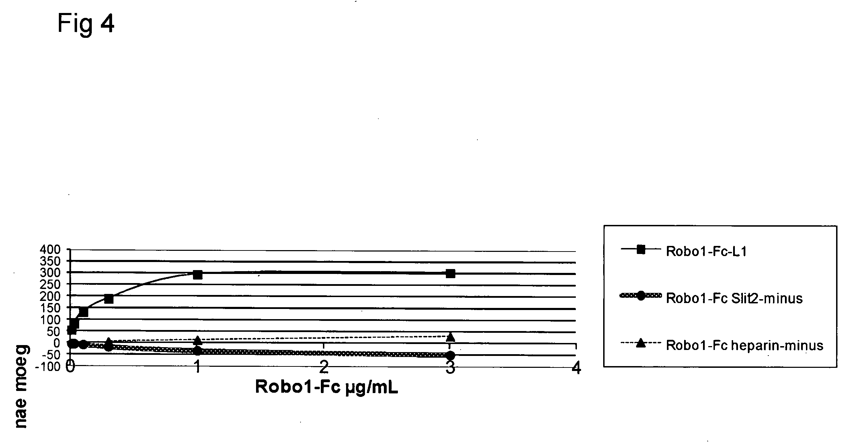

Figure 4 describes the binding of the HEK293 cells to the Robo1-Fc protein, via the fluorescence measured by FACS when Slit2-N is expressed by transient transfection. Only the Robo1-Fc protein binds to the HEK293 cells expressing Slit2-N. The Robo1-Fc Slit2-minus and Robo1-Fc heparin-minus mutants do not bind (or virtually not) in the Robo1-Fc concentration range of 3.0 to 0.3 mg/L, compared with the biotherapeutic Robo1-Fc L1 protein.

Table 7 describes the affinity constants measured by FACS for the Robo1-Fc protein when the Slit2-N, Slit2-D1D2 or Slit2-D2 proteins are expressed in the HEK293 line.

**Table 7: Affinity of Robo1-Fc for the Slit2 protein on cells by FACS**

| **Ligand Robo1-Fc** | **Protein expressed transiently in HEK293** | **K_{D} (nM)** |
|---|---|---|
| Robo1-Fc L1 | Slit2-N | 1.2 |
| | Slit2-D1D2 | 0.98 |
| | Slit2-D2 | Very weak binding |
| Robo1-Fc Heparin-minus | Slit2-N | 43 |
| | Slit2-D1D2 | 41 |
| Robo1-Fc Slit2-minus | Slit2-N | No binding |

As in the previous examples, the Robo1-Fc Slit2-minus mutant proved to be Slit2-negative and the Robo1-Fc heparin-minus mutant has a weaker affinity for Slit2 than the biotherapeutic protein.

Robo1-Fc binds to Slit2-N and Slit2-D1D2 expressed by the HEK293 cells with comparable affinities which are better than the affinity with Slit2-D2.

### Example 4: Pharmacokinetic properties of the Robo1-Fc L1 and Robo1-Fc L2 proteins

This example describes the pharmacokinetic profile and the plasma concentration of the Robo1-Fc protein injected once in mice intravenously (iv).

Three Balb/C mice (for each time) were injected, via the caudal vein, with each of the Robo1-Fc proteins at 2.5 mg/mL in a proportion of 100 µL/10 g (≈ 25 mg/kg). At the predetermined times (0.5, 1, 6, 24, 48 and 72 h after administration), the mice were anaesthetized, and blood was sampled and collected in a tube containing 10 µL of citrate (CPD-A, C-4431 Sigma) and 2 µL of protease inhibitors (Complete Protease Inhibitor Mix, Roche Molecular Biochemical). The tubes were centrifuged and the plasma samples were collected and frozen at -20°C.

The wells of 96-well plates were coated with the Slit2 protein (Slit2-D2), and the plasma samples, diluted to 1/5000 and 1/20 000, were brought into contact for one hour at 37°C. The peroxidase-conjugated anti-human Fc antibody (ref. 31413, Pierce) was subsequently incubated and then visualized with TMB-H₂O₂ (ref UP664780, Interchim) and the absorbance was read at 450 nm. A calibration range was prepared with each purified Robo1-Fc protein.

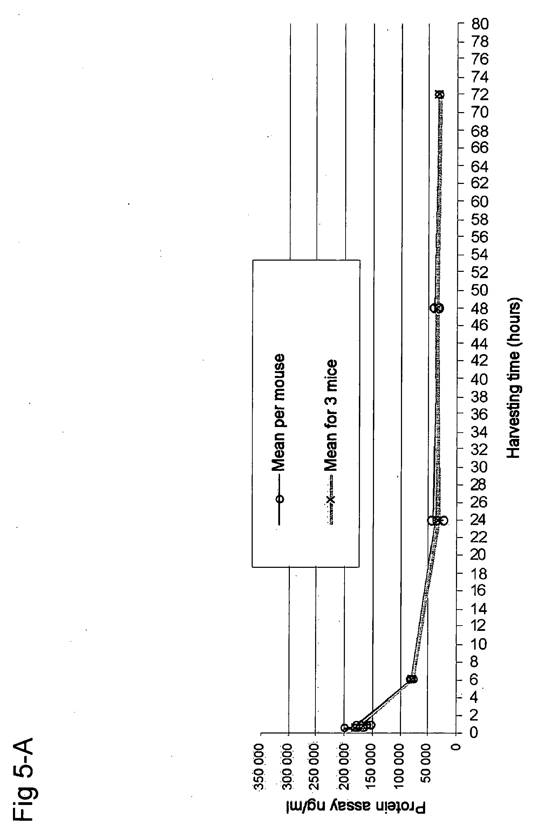

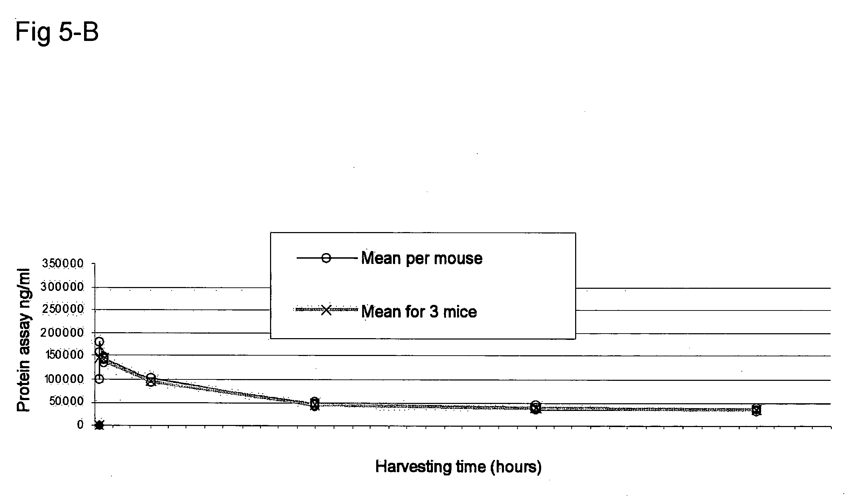

The plasma concentrations of the Robo1-Fc L1 and Robo1-Fc L2 proteins are represented in Figure 5. The pharmacokinetic parameters are described in the following table 8 and show that the protein is stable after injection in mice.

**Table 8: Pharmacokinetic parameters of the Robo1-Fc proteins after iv injection in mice**

| **Protein at 25 mg/kg** | **C₀ µg/mL** | **T_{1/2} h** | **AUC (0-last) µg.h/mL** | **AUC (0-24h) µg.h/mL** | **Cl mL/H/kg** | **Vdss mL/kg** |
|---|---|---|---|---|---|---|
| Robo1-Fc L1 | 203 | 158 | 3655 | 1879 | 2.2 | 470 |
| Robo1-Fc L2 | 198 | 142 | 3973 | 2042 | 2.2 | 417 |

### Example 5: Description of the Robo1-Fc biotherapeutic protein improved with respect to its homogeneity in the N-terminal position

This example describes the expression plasmid, the production and the physicochemical characterization of another Robo1-Fc protein, called Robo1-Fc L3, which is different from the Robo1-Fc L1 protein by virtue of the absence of the first two residues Ser20 and Arg21.

The cDNA cloned into the pXL4904 plasmid was modified by PCR in order to eliminate the Ser20 and Arg21 codons, and to fuse the next codon (Leu22) to the coding sequence of the peptide signal of interleukin 2. The pXL5004 expression plasmid was then generated, see Figure 1. The cDNA sequence used to express this recombinant protein corresponds to the sequence SEQ ID NO.23.

The Robo1-Fc L3 protein was produced, purified and characterized as described in example 1. The N-terminal analysis showed that this purified protein was perfectly homogeneous. The recombinant protein obtained is called Robo1-Fc L3 and corresponds to the protein sequence SEQ ID NO.24.

### Example 6: Evaluation of the interaction of the Robo1-Fc L3 protein with the human Slit2 protein

This example describes the interaction of the Robo1-Fc L3 fusion protein with the human Slit2 protein (Slit2-D2) by ELISA assay.

The human Slit2-D2 protein was bound to Immulon-4 enzyme-linked plates (VWR Scientific Inc. Swedesboro, NJ). A concentration range (from 1 µg/mL to 0.001 µg/mL) of the Robo1-Fc L3 fusion protein was added and then detected by means of the peroxidase-conjugated goat anti-human IgG antibody (Sigma; ref. A0170, dilution to 1:50 000). The visualization was carried out with the TMB-H₂O₂ substrate (Interchim; ref UP664780) and the measurements were carried out with a plate reader at 450 nm. The results obtained are reported in table 9 below.

**Table 9: Affinity of the Robo1-Fc L3 protein for the Slit2 protein - Comparison with the Robo1-Fc L1 protein**

| **Proteins studied** | **EC50 (µg/ml)** | **CV (%)** |
|---|---|---|
| Robo1-Fc L1 | 0.13 | 3.3 |
| Robo1-Fc L3 | 0.17 | 4.4 |

These results show that the affinities of the two variants Robo1-Fc L1 and Robo1-Fc L3 for the Slit2-D2 protein are comparable.

### Example 7: Evaluation of the activity of the Robo-Fc protein on neovascularization

### a. In vitro endothelial cell and fibroblast coculture model: specific activity of the Robo1-Fc L1 molecule

The *in vitro* angiogenesis model corresponds to a rearrangement of human vein endothelial cells on a monolayer of human dermal fibroblasts. Briefly, the fibroblasts (Lonza) are seeded into 24-well plates (Becton Dickinson) at 40 000 cells/well in 1 ml of medium. After 3 days of proliferation (D3), human vein endothelial cells (HUVEC-Lonza) are seeded onto the fibroblast cell monolayer at 10 000 cells/well in 500 µl of EGM^{®} medium (endothelial basal medium, Lonza) + 2% FCS (foetal calf serum - Lonza) + 10 µg/ml hEGF (recombinant human epidermal growth factor - Lonza). The cells are stimulated with 30 ng/mL of VEGF (R&D Systems), with the Robo1-Fc L1 molecule or with a Robo1-Fc Slit2-minus negative control at the concentration of 500 µg/ml (D3 to D9). After 3 days, the medium is replaced and the wells are stimulated according to the conditions of the experiment.

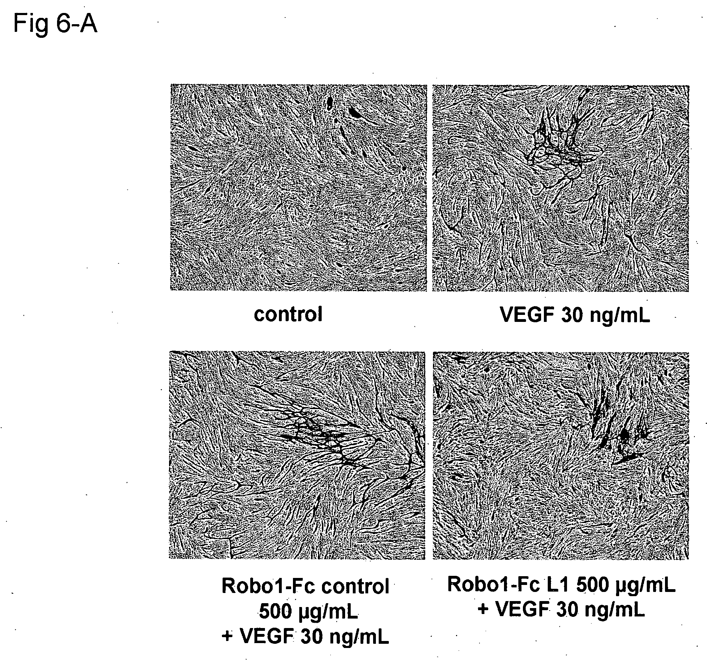

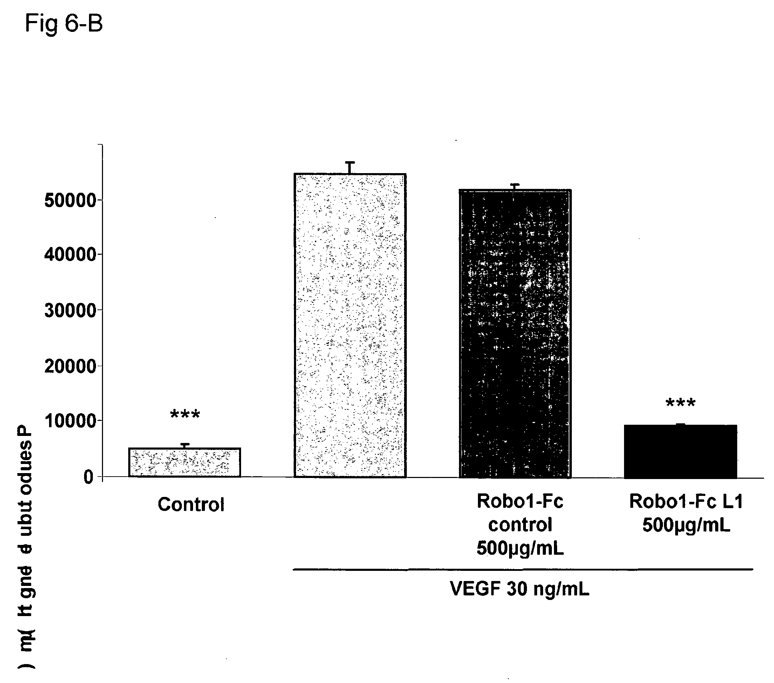

After 2 days, the cells are fixed with ethanol and labelled with an anti-CD31 antibody specific for HUVECs, followed by an anti-alkaline phosphatase antibody, and then visualized with an alkaline phosphatase substrate (D11). A quantification of the tubules labelled with the anti-CD31 antibody is carried out by means of image acquisitions made under a microscope (x4 objective) and the length of the pseudotubules is analysed using image analysis software (Biocom Visiolab 2000 software) (Figure 6).

In this *in vitro* angiogenesis assay, Robo1-Fc L1 (500 µg/ml) shows an inhibitory activity on the formation of the tubules formed by the HUVECs. This inhibition amounts to 82% and it is statistically significant compared with the effect obtained with the Robo1-Fc Slit2-minus molecule (negative control).

### b. Ex vivo mouse aortic ring model: specific activity of the Robo1-Fc L1 molecule

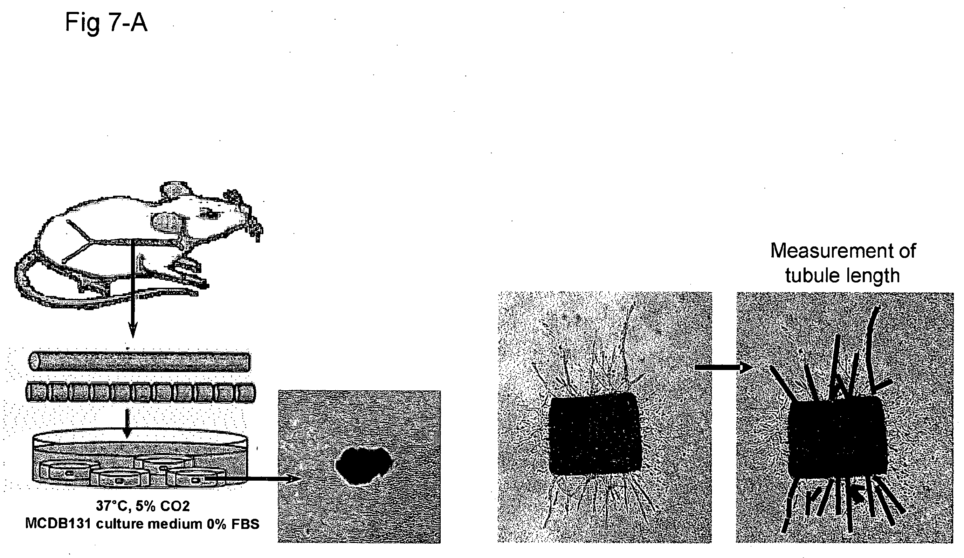

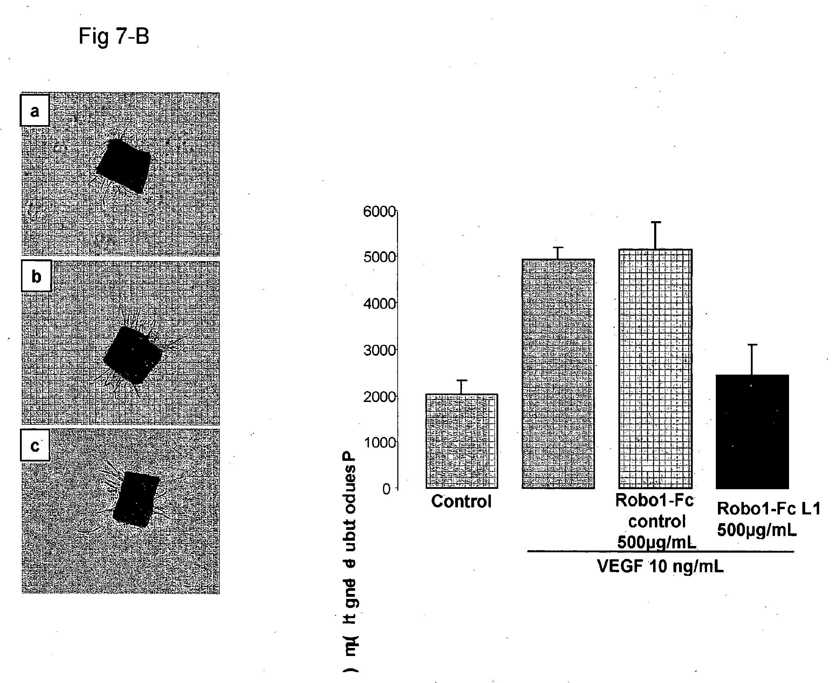

The Robo1-Fc L1 molecule was evaluated in a mouse aortic ring model. Briefly, mouse aortas are removed and cleaned, and cut into sections of 1 mm (D0). These rings are embedded in rat collagen in the presence of VEGF at 10 ng/ml, of the Robo1-Fc L1 molecule at the concentration of 500 µg/ml or of a Robo1-Fc Slit2-minus negative control at the concentration of 500 µg/ml. Tubules will form from the ring, thus mimicking *in vitro,* the formation of neovessels. After 6 days, a quantification of the labelled tubules is carried out by means of image acquisitions made under a microscope (x3 objective) (Figure 7A) and the length of the pseudotubules is analysed using image analysis software (Biocom Visiolab software 2000) (Figure 7).

Under these experimental conditions, Robo1-Fc L1 (500 µg/ml) shows a strong inhibitory activity on the formation of the tubules formed, in comparison with the Robo1-Fc Slit2-minus molecule used as a negative control.

These results suggest that Robo1-Fc L1 is capable of inhibiting the formation of neovessels without inhibiting endothelial cell proliferation. This anti-angiogenic activity linked to a vessel maturation defect is called "non-productive angiogenesis".

### Example 8: Evaluation of the Robo1-Fc L1 protein in a lung tumour model in mice

The Robo1-Fc L1 molecule was evaluated in a model of a lung cancer tumour in C57/BI6 mice.

### a. Murine lung tumour model

In order to establish the murine lung tumour model, 8-week-old female C57/BI6 mice were anaesthetized. The area at the level of the left scapula of the mouse was shaved and disinfected. A 1 cm incision was made above the scapula.

The cells to be injected are derived from a Lewis lung carcinoma tumour line (ATCC, CRL-1642). The cells were mixed with Matrigel^{®} in a ratio of 1 vol of Matrigel to 4 vol of cells. The cell concentration was 62 500 cells/ml. The cells were injected into the lung at a rate of 20 µl per mouse, and then the wound was sutured.

After 23 days, the mice were euthanized. The ribcage was opened up, and the left lung and the mediastinal lymph nodes were removed. The tumour present on the left lung was measured using an electronic calliper rule in order to determine the tumour volume according to the formula: I2xLx0.52. The mediastinal lymph nodes are weighed. The results are expressed as mean value ± standard deviation from the mean. The statistical analysis was carried out by means of a parametric Student's test.

### b. Treatment of the mice bearing a lung tumour with the Robo1-Fc recombinant protein

The treatment using the Robo1-Fc protein was carried out as follows: a preparation containing the Robo1-Fc protein was injected at the dose of 25 mg/kg/day, intravenously, on D10, D14, D17 and D21 post-injection of the tumour cells. The control group was injected with PBS buffer (10 ml/kg).

### c. Results

On D23, the mean volume of the tumours obtained in the group treated with the Robo1-Fc recombinant protein was 21.45 ± 2.16 mm³; the mean volume of the tumours obtained in the control group was 39.93 ± 8.41 mm³. The reduction in tumour volume in the animals treated with the Robo1-Fc protein is 46%. This difference is statistically significant (p<0.05). The mean weight of the mediastinal lymph nodes (metastatic lymph nodes) obtained in the group treated with the Robo1-Fc protein is 12.50 ± 1.26 mg. The mean weight of the mediastinal lymph nodes obtained in the control group is 30.67 ± 7.69 mg. The reduction in weight of the mediastinal lymph nodes for the group treated with the Robo1-Fc protein is 59% at the limit of significance (p=0.07).

### Example 9: Evaluation of the Robo1-Fc L1 protein in two models of human hepatocarcinoma

The Robo1-Fc L1 molecule was evaluated in two models of human hepatocarcinoma in SCID mice.

### A. Orthotopic model of human hepatocarcinoma in SCID mice using HepG2 cell line

### a. Description of the model

Female SCID mice are anaesthetized (with a mixture of ketamine/xylazine) and the area of the incision is shaved. The skin is cleaned and disinfected before making a 1 cm subcostal incision in the skin and in the muscle wall. A part of the lobe of the liver is carefully extracted from the peritoneum in order to carry out the injection of the cells. A 50 µl volume of the cell suspension (HepG2 originating from ATCC, human hepatoblastoma cells mixed with Matrigel ® at 40 x 10⁶ cells/ml) is injected into the left lobe. The lobe is put back into the abdominal cavity. The peritoneum is closed with surgical glue and then the skin is sutured with staples. The mice are treated with Robo1-Fc solubilized in PBS at 25 mg/kg, i.p. injection twice a week. The treatment begins two weeks after the implantation of the cells. Four weeks after inoculation with the cells, the animals are sacrificed for autopsy.

### b. Treatment of the mice bearing a human hepatocarcinoma with the Robo1-Fc recombinant protein

Robo1-Fc was evaluated in this model with a treatment twice a week at the dose of 25 mg/kg. The weight of the mice is measured on the first and last day of the protocol. The weight of the tumours was measured 28 days after the injection of the cells, after sacrificing the animals.

### c. Results

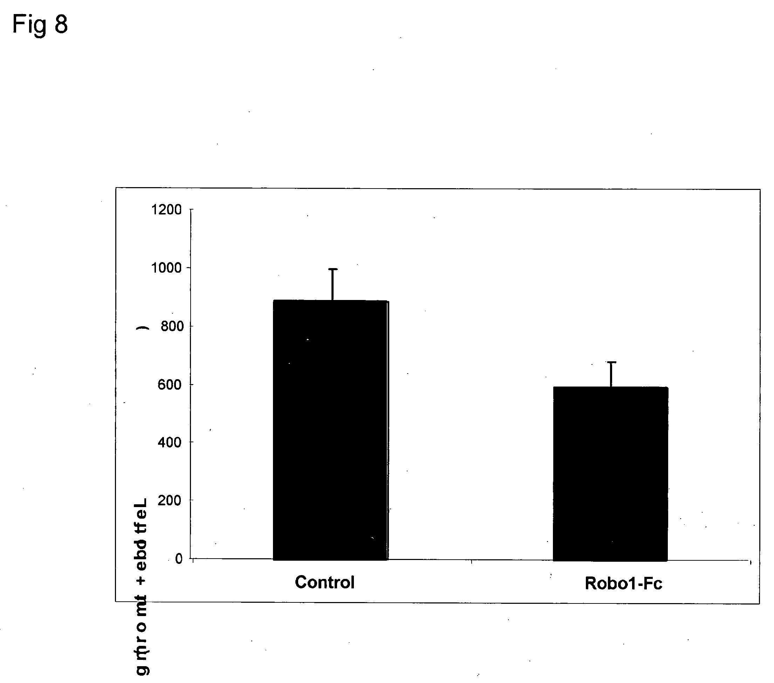

The tumour growth leads to a considerable loss of weight by the control animals during the protocol, whereas the animals treated with Robo1-Fc have an identical weight at the end of the experiment (Table 10). At the end of the experiment, after 4 i.p. injections of Robo1-Fc, the mean tumour weight of the Robo-1-Fc-treated group is significantly reduced by 30% (p < 0.05) in comparison with the control group having received injections of solvent (Figure 8).

**Table 10: Weight of animals at the beginning and end of the protocol**

| Mean weight of mice (g) | | |
|---|---|---|
| Mean | | |
| (+/- standard deviation of the mean) | D0 | D28 |
| Control (n=11) | 19.24 ± 0.48 | 16.46 ± 0.54 |
| Robo1-Fc (n=13) | 19.17 ± 0.31 | 19.07 ± 0.63 |

### B. Orthotopic model of human hepatocarcinoma in SCID mice using Hep3B cell line

### a. Description of the model

In this HCC model, Hep3B cell line was used. This tumor cell line is a commercial cell line (from ATCC) coming from an hepatoblastoma of a young patient. The same protocol of cell injection was used as previously described in example 9 A a). The initial concentration of cells is 2.10⁶ in an injected volume of 50µl.

### b.Treatment of mice bearing Hep3B tumors with Robo1-Fc

Robo1-Fc was evaluated in this model by a treatment twice a week at 5 mg/kg. The mice were weighted before treatment and each time before the next treatment in order to adjust the dosage with the injected volume. The mice were also weighted the last day of the protocol, before sacrifice. The lobe with the tumor was measured after sacrifice on the last day of the protocol. Furthermore, before sacrifice, the blood of mice were withdrawn and the serum was discarded for αfetoprotein (AFP) determination; AFP is secreted by the cancer liver cells and it is currently accepted as a biomarker of HCC development in humans (Fimus et al., 2004, Mol Diagn8(4):207-12. *Review).*

### c. Results

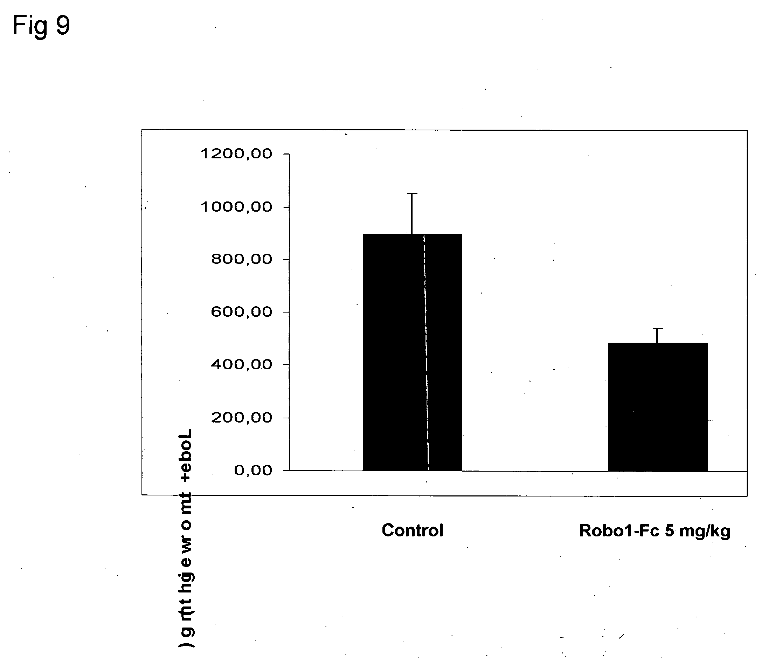

The mice did not gain weight during the tumoral development for 35 days. The treatment of Robo1-Fc at 5 mg/kg showed no effect on the mice weight (Table 11). At the end of the experiment, the mean tumoral weight of the control group was 895 ±156 mg. the mean tumoral weight of the Robo1-Fc treated group was 483±55 mg (Figure 9). Treatment with Robo1-Fc induced a significant reduction of the tumor burden of 46%.

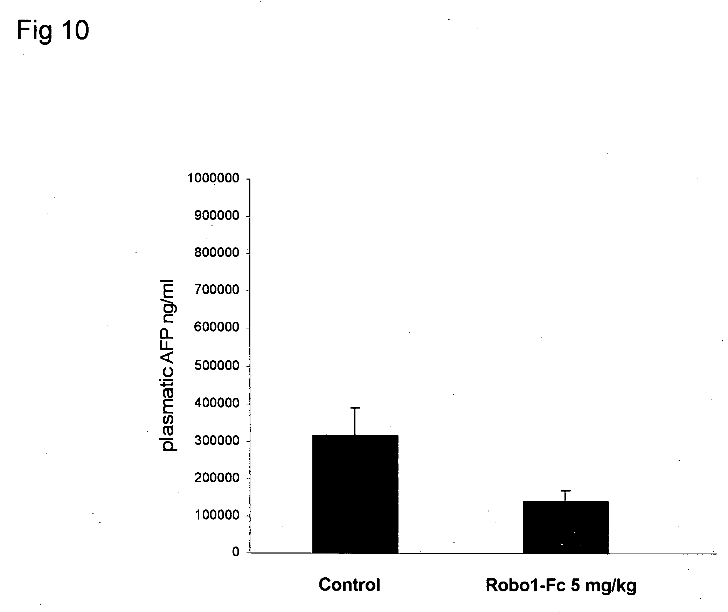

Furthermore the plasmatic AFP concentration was 316 ± 73 µg/ml in control tumor bearing mice and 139 ± 28 µg/ml in Robo1-Fc treated mice, showing a 56 % inhibition of plasmatic AFP in treated animals (Figure 10). This inhibition of plasmatic AFP correlated well with the anti tumoral activity of Robo1-Fc.

**Table 11 : Mice weight at the beginning and at the end of the protocol**

| Mice weight | D0 | D35 |
|---|---|---|
| Mean +/- sem | | |
| Control group (n=14) | 19.08 ± 0.30 | 19.12 ± 0.76 |
| Robo1-Fc group (n=14) | 19.36 ± 0.36 | 20.37 ± 0.31 |

## Claims

1. Recombinant protein comprising the extracellular domain derived from Robo1 protein or a part of this domain, a linker and an immunoglobulin Fc domain, for use in the treatment of hepatocarcinoma.

2. Recombinant protein for use according to Claim 1, wherein the recombinant protein inhibits angiogenesis without inhibiting endothelial cell proliferation.

3. Recombinant protein for use according to Claim 1 or 2, wherein said extracellular domain is an extracellular domain derived from isoform b of the Robo1 protein.

4. Recombinant protein for use according to Claim 3, in which the extracellular domain of Robo1 or part thereof consists in the Ig1 and Ig2 immunoglobulin domains of said extracellular domain.

5. Recombinant protein for use according to Claim 4, in which the extracellular domain of Robo1 has at least 80% identity with the sequence SEQ ID NO.2,

6. Recombinant protein for use according to any one of Claims 1 to 5, in which the Fc domain comes from human immunoglobulin G4.

7. Recombinant protein for use according to Claim 6, in which the Fc domain contains the S241P and L248E mutations and in which the lysine located in the C-terminal position is absent.

8. Recombinant protein for use according any one of the preceding Claims, the sequence of which has at least 80% identity with the sequence SEQ ID NO. 4, SEQ ID NO.6 or SEQ ID NO. 24.

9. Recombinant protein for use according any one of the preceding Claims, the sequence of which is the sequence SEQ ID NO. 4, SEQ ID NO.6: or SEQ ID NO. 24.

## Patentansprüche

1. Rekombinantes Protein, umfassend die von Robo1-Protein stammende extrazelluläre Domäne oder einen Teil dieser Domäne, einen Linker und eine Immunglobulin-Fc-Domäne, zur Verwendung bei der Behandlung von Hepatokarzinom.

2. Rekombinantes Protein zur Verwendung nach Anspruch 1, wobei das rekombinante Protein Angiogenese hemmt, ohne die Endothelzellproliferation zu hemmen.

3. Rekombinantes Protein zur Verwendung nach Anspruch 1 oder 2, wobei die extrazelluläre Domäne ist eine extrazelluläre Domäne von Isoform b Robo1 Protein abgeleitet.

4. Rekombinantes Protein zur Verwendung nach Anspruch 3, bei dem die extrazelluläre Domäne von Robo1 oder ein Teil davon in den Ig1- und Ig2-Immunglobulindomänen der extrazellulären Domäne besteht.

5. Rekombinantes Protein zur Verwendung nach Anspruch 4, bei dem die extrazelluläre Domäne von Robo1 wenigstens 80% Identität mit der Sequenz SEQ ID NO. 2 aufweist.

6. Rekombinantes Protein zur Verwendung nach einem der Ansprüche 1 bis 5, bei dem die Fc-Domäne aus menschlichem Immunglobulin G4 stammt.

7. Rekombinantes Protein zur Verwendung nach Anspruch 6, bei dem die Fc-Domäne die Mutationen S241 P und L248E enthält und bei dem das Lysin in der C-terminalen Position fehlt.

8. Rekombinantes Protein zur Verwendung nach einem der vorhergehenden Ansprüche, dessen Sequenz wenigstens 80% Identität mit der Sequenz SEQ ID NO. 4, SEQ ID NO. 6 oder SEQ ID NO. 24 aufweist.

9. Rekombinantes Protein zur Verwendung nach einem der vorhergehenden Ansprüche, bei dessen Sequenz es sich um die Sequenz SEQ ID NO. 4, SEQ ID NO. 6 oder SEQ ID NO. 24 handelt.

## Revendications

1. Protéine recombinante comprenant le domaine extracellulaire dérivé de la protéine Robo1 ou une partie de ce domaine, un agent de liaison et un domaine de Fc d'immunoglobuline, destinée à une utilisation dans le traitement d'un hépatocarcinome.

2. Protéine recombinante destinée à une utilisation selon la revendication 1, la protéine recombinante inhibant l'angiogenèse sans inhiber la prolifération des cellules endothéliales.

3. Protéine recombinante destinée à une utilisation selon la revendication 1 ou 2, dans laquelle ledit domaine extracellulaire est un domaine extracellulaire dérivé d'une isoforme b de la protéine Robo1.

4. Protéine recombinante destinée à une utilisation selon la revendication 3, dans laquelle le domaine extracellulaire de Robo1 ou une partie de celui-ci est constitué des domaines d'immunoglobuline Ig1 et Ig2 dudit domaine extracellulaire.

5. Protéine recombinante destinée à une utilisation selon la revendication 4, dans laquelle le domaine extracellulaire de Robo1 présente une identité de séquence d'au moins 80 % vis-à-vis de la séquence SEQ ID N° 2.

6. Protéine recombinante destinée à une utilisation selon l'une quelconque des revendications 1 à 5, dans laquelle le domaine de Fc provient d'une immunoglobuline G4 humaine.

7. Protéine recombinante destinée à une utilisation selon la revendication 6, dans laquelle le domaine de Fc contient les mutations S241 P et L248E et dans laquelle la lysine située dans la position C-terminale est absente.

8. Protéine recombinante destinée à une utilisation selon l'une quelconque des revendications précédentes, dont la séquence présente une identité d'au moins 80 % vis-à-vis de la séquence SEQ ID N°4, SEQ ID N°6 ou SEQ ID N°24.

9. Protéine recombinante destinée à une utilisation selon l'une quelconque des revendications précédentes, dont la séquence est la séquence SEQ ID N°4, SEQ ID N°6 ou SEQ ID N°24.
